(19) Europäisches Patentamt
European Patent Office
Office européen des brevets

(11) **EP 4 620 549 A1**

(12) **EUROPEAN PATENT APPLICATION**
published in accordance with Art. 153(4) EPC

(43) Date of publication:
**24.09.2025  Bulletin 2025/39**

(51) International Patent Classification (IPC):
**B01D 29/01** (2006.01)  **C12N 1/00** (2006.01)
**C12M 1/28** (2006.01)

(21) Application number: **23891247.1**

(22) Date of filing: **12.10.2023**

(52) Cooperative Patent Classification (CPC):
**B01D 29/01; C12M 1/28; C12N 1/00**

(86) International application number:
**PCT/JP2023/037068**

(87) International publication number:
**WO 2024/106089 (23.05.2024 Gazette 2024/21)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **14.11.2022   JP 2022181802**

(71) Applicant: **Tokyo Ohka Kogyo Co., Ltd.
Kawasaki-shi, Kanagawa 211-0012 (JP)**

(72) Inventors:
• **OHSAKA, Takashi**
  **Kawasaki-shi, Kanagawa 211-0012 (JP)**
• **TAKAHASHI, Anna**
  **Kawasaki-shi, Kanagawa 211-0012 (JP)**
• **NAKAMURA, Akimasa**
  **Kawasaki-shi, Kanagawa 211-0012 (JP)**

(74) Representative: **Plasseraud IP
104 Rue de Richelieu
CS92104
75080 Paris Cedex 02 (FR)**

(54) **WELL ARRAY FILTER, PARTICLE ALIGNMENT DEVICE, AND PARTICLE CAPTURE METHOD**

(57)    In a well array filter (1), a plurality of wells (3) are formed in a filter body (2), the wells (3) adjacent to each other are separated by a well partition wall (6), two or more through-holes (8) are formed in a well bottom portion (4), the minimum width of the opening of the through-hole (8) is 1 μm or more and 3.5 μm or less, and the ratio of a total opening area of the through-hole is 0.5% or more and 3.5% or less. Assuming a minimum circumscribed circle (G1) that surrounds all of the through-holes (8) in each well bottom portion (4), when the center is moved to the center of each of the plurality of through-holes (8), all minimum circumscribed circles (G2) after movement at least partially overlap all of the plurality of through-holes (8).

FIG. 3

EP 4 620 549 A1

**Description**

TECHNICAL FIELD

The present invention relates to a well array filter for capturing particles such as cells or beads, a particle alignment device, and a particle capture method.

Priority is claimed on Japanese Patent Application No. 2022-181802, filed November 14, 2022, the content of which is incorporated herein by reference.

BACKGROUND ART

In recent years, particularly in the field of drug discovery, the target of cell analysis is subdivided from a cell group level to a single cell level, a cell screening device (particle alignment device) is used to capture cells one by one in a large number of fine wells of a well array filter, a screening test is then performed on a large number of cells at once, and cells having desired characteristics are selected. As cell screening methods, for example, a method in which cells captured in a large number of wells are brought into contact with a liquid in which a reagent such as a catcher that binds to a specific antibody is dispersed, and cells that have secreted a secretion bound to the catcher are found, and collected from the wells is used.

The particle alignment device disclosed in Patent Document 1 includes a well array filter made of a resist, and in the well array filter, a large number of wells, each having one through-hole in the bottom portion, are formed in an array. The opening shape of each well is a circle or ellipse, resembling a cell. In examples of Patent Document 1, a well array filter in which the opening diameter of the well is 100 $\mu$m, the depth of the well is 30 to 100 $\mu$m, and the opening diameter of the through-hole is 2 to 5 $\mu$m is disclosed.

The particle alignment device disclosed in Patent Document 2 includes a well array filter made of a silicon wafer, and in the well array filter, a large number of wells are formed in an array by etching, and a SiNx film having one through-hole formed therein is provided in the bottom portion of each well. The opening shape of each well is a circle. This well array filter is made of a silicon wafer, and in examples of Patent Document 2, the thickness of the filter body is 380 $\mu$m, the opening diameter of the well is about 100 $\mu$m, and the distance between the centers of the wells is about 150 $\mu$m.

The particle alignment device disclosed in Patent Document 3 includes a well array filter for secretion assay, which is formed of two layers: a well substrate having a through-hole in the bottom portion for storing cells and a substrate for storing cells for detecting secretions from the cells. The well substrate is made of a resist. In examples of Patent Document 3, the wells are circular and have an opening diameter of 12 $\mu$m, which is a size close to that of cells, and the distance between the centers of the wells is about 100 $\mu$m.

CITATION LIST

Patent Document

Patent Document 1: US Patent No. 10370630
Patent Document 2: US Patent No. 9638636
Patent Document 3: Japanese Unexamined Patent Application, First Publication No. 2019-213566

SUMMARY OF INVENTION

Technical Problem

By the way, in the above well array filters, when a sample liquid in which cells, beads and the like are dispersed in a dispersion medium is passed through the well array filter, it is preferable that cells and the like be captured in as many wells as possible, and cells and the like be captured so that only one cell is stored, i.e., a single cell, is stored in one well. Cells and the like can be captured in many wells in one passing, but when the single cell rate is high, it is possible to separate target cells and the like one by one and capture them with a high probability.

On the other hand, when cells are captured and then cultured or when cells and the like are collected from individual wells using a capillary, it is easier to handle them if the opening diameter of the well is large to a certain extent. This is because, when cells are cultured in wells with a small opening diameter, the divided cells may overflow from the wells and may not be captured, and furthermore, if the opening diameter of the well is larger than the outer diameter of the tip of the capillary, the tip of the capillary can be directly inserted into the well, and cells and the like can be easily collected.

However, if the opening diameter of the well simply increases, the number of wells per unit area of the well array filter is reduced. For example, if the width of the wall separating the wells is set to be constant, when comparing the case in which the opening diameter of the well is 20 $\mu$m and the case in which the opening diameter of the well is 50 $\mu$m, the number of wells per unit area becomes 1/4. If the opening diameter of one of the through-holes formed in the well bottom portion is the same in both the case of 20 $\mu$m and the case of 50 $\mu$m, the ratio of the area of the through-hole per area of the filter, that is, the opening ratio of the through-hole, also decreases to 1/4, the liquid permeability of the entire well array filter deteriorates, and the efficiency of a liquid passing operation decreases.

If the opening diameter of one through-hole formed in each well is increased in order to improve the liquid permeability of the well array filter, there is a risk of cells and the like passing through the through-hole. Therefore, increasing the number of through-holes in each well is considered, but a phenomenon in which simply increasing the number of through-holes reduces the single cell rate even if the opening diameter of the well is the same has been found.

The inventors analyzed this phenomenon in detail, and found that, in order to increase the single cell rate while securing the liquid permeability of the well array filter, it is preferable to form through-holes to achieve the following effects. That is, (1) when cells and the like are captured in any one well, the cells and the like narrow the opening area of all the through-holes in the well, the total flow rate is reduced, the flow rate is not sufficient to guide the second cells and the like to the same well, and the cells and the like are guided to and captured in an adjacent empty well, (2) however, when a single cell or the like is captured in one well, if all the through-holes are blocked and the flow rate is extremely reduced, the liquid permeability of the entire well array filter deteriorates.

The inventors examined the conditions for the through-holes to achieve the above effects. As a result, it was found that, when two or more through-holes are formed in a well bottom portion, the minimum width of the upper-surface opening of the through-hole is 1 $\mu$m or more and 3.5 $\mu$m or less, the ratio of a total opening area of the through-holes to the area of the well formation region of the well array filter is 0.5% or more and 3.5% or less, and assuming a minimum circumscribed circle that surrounds all of the plurality of through-holes in the same well, if the center of the minimum circumscribed circle is moved to the center of any of the plurality of through-holes, when the minimum circumscribed circle satisfies a positional relationship in which it at least partially overlaps all of the plurality of through-holes, the above effects are obtained.

The present invention has been made in view of the above circumstances, and an object of the present invention is to provide a well array filter, a particle alignment device and a particle capture method through which particles such as cells can be captured one by one in as many wells as possible, the single cell rate is high, the liquid permeability is favorable, and a large number of cells and the like can be aligned and captured stably.

Solution to Problem

[Aspect 1] A well array filter according to Aspect 1 of the present invention has a flat filter body, and in the filter body, a plurality of open wells are formed in a well formation region on the upper surface of the filter body, and the wells adjacent to each other are separated by a well partition wall, a well bottom portion is formed at the lower end of the well, and in the well bottom portion, two or more through-holes that reach the lower surface of the filter body are formed, the minimum width of the upper-surface opening of the through-hole is 1 $\mu$m or more and 3.5 $\mu$m or less, the ratio of a total opening area of the through-holes to the area of the well formation region is 0.5% or more and 3.5% or less, and assuming a minimum circumscribed circle that surrounds all of the plurality of through-holes formed in each well bottom portion, when the center of the minimum circumscribed circle is moved to the center of each of the plurality of through-holes, the minimum circumscribed circle after movement satisfies a positional relationship in which the minimum circumscribed circle after movement at least partially overlaps all of the plurality of through-holes.

In the well array filter according to Aspect 1, the minimum width of the through-hole is 1 $\mu$m or more and 3.5 $\mu$m or less, the ratio of a total opening area of the through-holes to the area of the well formation region is 0.5% or more and 3.5% or less, and thus the flow rate of the entire well array filter can be secured while restricting particles to be captured such as cells or beads from passing through the through-hole. In addition, since the plurality of through-holes formed in each well bottom portion satisfy the positional relationship, when one particle is captured in the well, there is a high probability of the particle at least partially overlapping all of the plurality of through-holes. Therefore, after the first particle is captured, the flow rate of a dispersion medium that passes through all the through-holes in the well is appropriately reduced, the second and subsequent particles are restricted from entering the same well according to the flow, and there is a high probability of cells and the like being guided to and captured in an adjacent empty well. Therefore, there is a high probability of particles being captured one by one in many wells, the single cell rate is high, favorable liquid permeability is maintained without any decrease, and isolation of particles in each well is stably performed.

[Aspect 2] In Aspect 1, regarding the through-holes formed in each well bottom portion, two or more through-holes may be formed on an imaginary line segment, a total of three or more through-holes may be formed at vertex positions of an imaginary triangle and on the sides or inside of the triangle, or a total of four or more through-holes may be formed at vertex positions of an imaginary quadrangle and on the sides or inside of the quadrangle.

In this case, when one particle such as a cell or a bead is captured in the well, there is a high rate of the particle at least partially overlapping all of the plurality of through-holes. Therefore, after the first particle is captured, the flow rate of a dispersion medium that passes through all the through-holes in the well is appropriately reduced, the second and subsequent particles are restricted from entering the well, and thus isolation of particles in each well is more stably performed.

[Aspect 3] In Aspect 1 or 2, the opening shape of the through-hole may be a circle, an ellipse, or a shape in which a plurality of circles or ellipses are connected.

In this case, since the opening shape of the through-hole is a circle, an ellipse, or a shape in which a plurality of circles or ellipses are connected, when a particle such as a cell or a bead is placed on the through-hole, there is a high rate of the particle at least partially overlapping all of the plurality of through-holes, a large difference in flow rate before and after the particle is captured in the well can be secured, and the effects can be enhanced.

[Aspect 4] In any one of Aspects 1 to 3, the opening shape of the through-hole may be a polygonal shape or a polygonal shape with rounded corners. The polygonal shape may be a triangular shape, a rectangular shape, or a pentagonal shape.

In this case, when the opening shape of the through-hole is a polygonal shape or a polygonal shape with rounded corners, since the minimum width of the upper-surface opening of the through-hole is small for the cross-sectional area of the flow path of the through-hole, it is possible to further restrict particles to be captured such as cells or beads from passing through the through-hole. In addition, since the well has a polygonal shape, it is easy to distinguish the particles from the well during automatic image analysis.

[Aspect 5] In any one of Aspects 1 to 4, a polymer that restricts cell adhesion may be applied to at least the inner surface of the well. The polymer may be, for example, an MPC polymer, PEG, PVA, PMEA, or a mixture thereof.

In this case, since the polymer applied to the inner surface of the well restricts cell adhesion, it is easy to remove the cells or particles from the well. The polymer may be applied not only to the inner surface of the well but also to the upper surface of the well partition wall, the inner surface of the through-hole, and the lower surface of the filter body. In this case, it is possible to reduce problems such as of cells and the like to the upper surface of the well partition wall, and adhesion of cells and the like to the lower surface of the filter body.

[Aspect 6] In any one of Aspects 1 to 5, the maximum inscribed circle diameter of the through-hole may be 4 μm or less.

In this case, it is possible to effectively restrict particles such as cells or beads from passing through the through-hole.

[Aspect 7] In any one of Aspects 1 to 6, the well partition wall and the well bottom portion may be formed of different materials, and the well bottom portion may be formed of a material having a higher strength than the well partition wall.

In this case, since the well bottom portion is formed of a material having a higher strength than the well partition wall, it is possible to increase the strength of the entire well array filter, mitigate shrinkage that occurs during molding of the well wall portion, and reduce warping and distortion of the well array filter.

[Aspect 8] In any one of Aspects 1 to 7, the well partition wall and the well bottom portion may be formed of different materials, the well bottom portion may be divided into tiles corresponding to the shapes of the openings of the wells, gaps may be formed between adjacent well bottom portions, and the material forming the well partition wall may penetrate into these gaps and solidify.

In this case, since the well bottom portion is divided into tiles, gaps are formed therebetween, the well partition wall is then formed, and the material forming the well partition wall can penetrate into the gaps and solidify, compared to when the well bottom portion is formed of a single plate, it is possible to reduce warping and distortion occurring in the well array filter due to solidification and shrinkage.

[Aspect 9] In any one of Aspects 1 to 8, the thickness of the well bottom portion may be 1 nm or more and 2 μm or less.

In this case, since the well bottom portion is thin, a captured object in the well can be easily observed through the well bottom portion under an inverted microscope, and since the amount of autofluorescence is small due to the thinness, the adverse effect of autofluorescence on observation can be reduced.

[Aspect 10] A particle alignment device according to Aspect 10 of the present invention includes the well array filter according to any one of Aspects 1 to 9, and a device body that supports the well array filter and has a sample flow path from the side of the opening of the well of the well array filter toward the bottom side of the well through the through-hole.

In the particle alignment device according to Aspect 10, the flow rate of the entire well array filter can be secured while restricting particles to be captured such as cells or beads from passing through through-holed. In addition, since the plurality of through-holes formed in each well bottom portion satisfy the positional relationship, when one particle is captured in the well, there is a high probability of the particle at least partially overlapping all of the plurality of through-holes. Therefore, after the first particle is captured, the flow rate of a dispersion medium that passes through all the through-holes in the well is appropriately reduced, the second and subsequent particles are restricted from entering the well according to the flow, and there is a high probability of cells and the like being guided to and captured in an adjacent empty well.

Therefore, there is a high probability of particles being captured one by one in many wells, the single cell rate is high, the liquid permeability is favorable, and isolation of particles in each well is stably performed.

[Aspect 11] A particle capture method according to Aspect 11 of the present invention includes a step of passing a liquid containing an organic solvent that does not dissolve or alter the well array filter and the device body through the sample flow path in the particle alignment device according to Aspect 10, a step of passing an aqueous solution through the sample flow path and replacing the liquid containing the organic solvent with the aqueous solution, and a step of passing an aqueous solution containing particles to be captured in the well through the sample flow path replaced with the aqueous solution and capturing the particles in the well. The liquid containing the organic solvent is not limited, and for example, a liquid such as ethyl alcohol or an aqueous solution containing an organic solvent such as about 30 to 80% of ethyl alcohol, and preferably an aqueous solution containing 70% of ethyl alcohol can be used.

In the particle capture method according to Aspect 11, when the liquid containing the organic solvent is passed through the sample flow path, the inside of the sample flow path is then replaced with an aqueous solution, and an aqueous solution containing particles such as cells, beads to which cell components or secretions are adhered, or beads for adhering cell components or secretions is then passed through, the liquid passage resistance of the well array filter is reduced, air bubbles are less likely to remain in the well, and it becomes easy to capture the cells or the particles one by one in each well.

[Aspect 12] In any one of Aspects 1 to 11, if the center of the minimum circumscribed circle is moved to the center of any of the plurality of through-holes, the minimum circumscribed circle at least partially may overlap all of the plurality of through-holes, and the maximum width of the overlapping portion having the smallest overlapping area among all of the overlapping portions may be 0.7% or more of the radius of the minimum circumscribed circle. The maximum width is a value along a straight line connecting the centers of the minimum circumscribed circle before and after the movement. The maximum width may be any one of 0.5% or more, 1% or more, 2% or more, 5% or more, 10% or more, 15% or more, 20% or more, and 30% or more of the radius of the minimum circumscribed circle. If the shape of the through-hole is a circle, when the maximum width of the overlapping portion is 0.7% of the radius of the minimum circumscribed circle, the overlapping area of the overlapping portion having the smallest overlapping area among all of the overlapping portions corresponds to 0.025% of the opening area of the through-hole. Similarly, when the overlapping area of the overlapping portion is 0.05%, 0.10%, 0.25%, 0.50%, and 1.00% of the opening area of the through-hole, the maximum width corresponds to 1.12%, 1.80%, 3.32%, 5.20%, and 8.26% of the radius of the minimum circumscribed circle. Even if the shape of the through-hole is any shape other than a circle, the overlapping area of the overlapping portion having the smallest overlapping area among all of the overlapping portions may be 0.025%, 0.05%, 0.10%, 0.25%, 0.50%, or 1.00% of the opening area of the through-hole.

Advantageous Effects of Invention

As described above, according to the present invention, since the flow rate of the entire well array filter can be secured while restricting particles to be captured such as cells or beads from passing through through-holed, and the plurality of through-holes formed in each well bottom portion satisfy the positional relationship, when one particle is captured in the well, there is a high probability of the particle at least partially overlapping all of the plurality of through-holes. Therefore, after the first particle is captured, the flow rate of a dispersion medium that passes through all the through-holes in the well is appropriately reduced, the second and subsequent particles are restricted from entering the well according to the flow, and there is a high probability of cells and the like being guided to and captured in an adjacent empty well. Therefore, there is a high probability of particles being captured one by one in many wells, the single cell rate is high, the liquid permeability is favorable, and isolation of particles in each well is stably performed.

BRIEF DESCRIPTION OF DRAWINGS

[FIG. 1] FIG. 1 is an enlarged plan view of a well array filter according to a first embodiment of the present invention.
[FIG. 2] FIG. 2 is an enlarged cross-sectional view of the well array filter according to the first embodiment.
[FIG. 3] FIG. 3(a) and FIG. 3(b) are enlarged plan views of the well of the well array filter according to the first embodiment, and FIG. 3(c) is an enlarged plan view of a well of a well array filter of a comparative example.
[FIG. 4] FIG. 4(a) to FIG. 4(c) are enlarged plan views of wells of well array filters according to a second embodiment to a fourth embodiment.
[FIG. 5] FIG. 5(a) to FIG. 5(c) are enlarged plan views of wells of well array filters according to a fifth embodiment to a seventh embodiment.
[FIG. 6] FIG. 6(a) and FIG. 6(b) are enlarged plan views of wells of well array filters according to an eighth embodiment and a ninth embodiment, and FIG. 6(c) is an enlarged plan view of a well of a well array filter of another comparative example.

[FIG. 7] FIG. 7(a) and FIG. 7(b) are enlarged plan views of wells of well array filters according to a tenth embodiment and an eleventh embodiment, and FIG. 7(c) is an enlarged plan view of a well of a well array filter of another comparative example.

[FIG. 8] FIG. 8(a) to FIG. 8(c) are enlarged plan views of a well of a well array filter of another comparative example.

[FIG. 9] FIG. 9(a) and FIG. 9(b) are enlarged plan views of wells of well array filters according to a twelfth embodiment and a thirteenth embodiment, and FIG. 9(c) is an enlarged plan view of a well of a well array filter of another comparative example.

[FIG. 10] FIG. 10(a) and FIG. 10(b) are enlarged plan views of wells of well array filters according to a fourteenth embodiment and a fifteenth embodiment, and FIG. 10(c) is an enlarged plan view of a well of a well array filter of another comparative example.

[FIG. 11] FIG. 11 is an enlarged plan view of a well array filter according to a sixteenth embodiment.

[FIG. 12] FIG. 12 is an enlarged plan view of a well array filter according to a seventeenth embodiment.

[FIG. 13] FIG. 13(a) to FIG. 13(f) are enlarged cross-sectional views illustrating a method of producing a well array filter according to an embodiment of the present invention.

[FIG. 14] FIG. 14 is a plan view showing a particle alignment device according to one embodiment of the present invention.

[FIG. 15] FIG. 15 is a longitudinal cross-sectional view showing the particle alignment device according to the same embodiment.

[FIG. 16] FIG. 16 is a diagram illustrating conditions for realizing examples of the present invention.

[FIG. 17] FIG. 17 is a diagram illustrating conditions for realizing examples of the present invention.

[FIG. 18] FIG. 18 is a graph illustrating conditions for realizing examples of the present invention.

[FIG. 19] FIG. 19 is a diagram illustrating conditions for realizing examples of the present invention.

[FIG. 20] FIG. 20 is a graph illustrating conditions for realizing examples of the present invention.

[FIG. 21] FIG. 21 is a table showing the effects of examples of the present invention.

[FIG. 22] FIG. 22 is a graph showing the effects of examples of the present invention.

[FIG. 23] FIG. 23 is a table showing the effects of examples of the present invention.

[FIG. 24] FIG. 24 is a graph showing the effects of examples of the present invention.

DESCRIPTION OF EMBODIMENTS

Embodiments of the present invention will be described below with reference to the drawings.

[First embodiment]

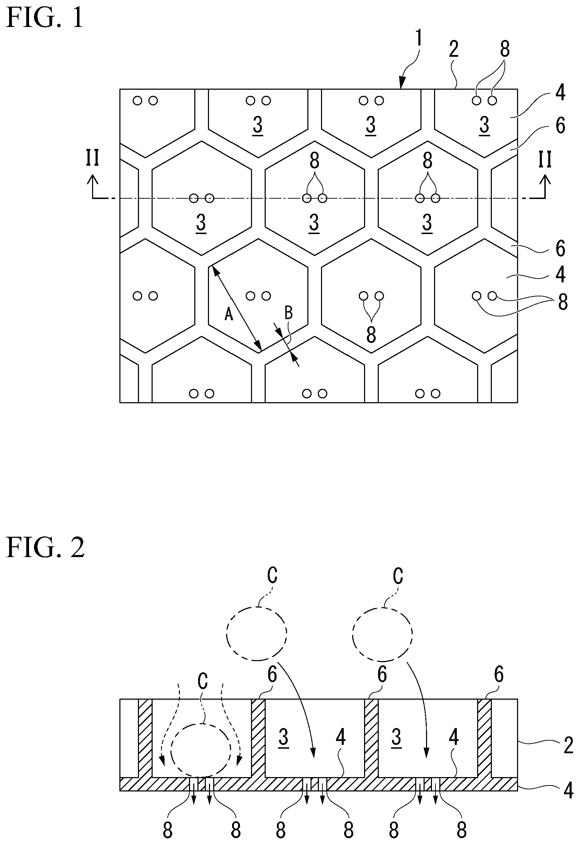

FIG. 1 and FIG. 2 are an enlarged plan view and an enlarged cross-sectional view showing a well array filter according to a first embodiment. A well array filter 1 has a flat filter body 2 with a certain thickness, and in the filter body 2, a large number of open wells 3 are formed in a well formation region on the upper surface of the filter body 2. The well formation region is a region of the filter body 2, which is used as a filter, and the filter body 2 may have a support region in which no wells are formed, which is for supporting the filter, around the well formation region.

The wells 3 adjacent to each other are separated by well partition walls 6. At the lower end of each well 3, as shown in FIG. 2, a flat well bottom portion 4 is formed, and blocks the lower end of the well 3. The opening of the well 3 may have a circular shape or an elliptical shape, but in a preferable embodiment, the opening has a polygonal shape or a polygonal shape with rounded corners. Particularly, in this embodiment, the wells 3 are formed in a regular hexagonal shape, and the wells 3 and the well partition walls 6 are arranged in a honeycomb pattern. At least the well bottom portion 4 is formed of a transparent material so that particles such as cells C in the well 3 can be observed through the well bottom portion 4 under an inverted microscope. The well partition wall 6 may also be formed of a transparent material, but it can also be formed of an opaque material.

The equivalent circle diameter of the opening of the well 3 is not limited, and is preferably 5 $\mu$m or more and 200 $\mu$m or less. The equivalent circle diameter is the diameter of a circle having the same area as the opening of the well 3. When the equivalent circle diameter of the opening of the well 3 is 5 $\mu$m or more, it becomes possible to capture particles such as cells or beads, and when the equivalent circle diameter is 200 $\mu$m or less, it becomes easy to capture particles such as cells or beads one by one or close to certain numbers in each well 3. The equivalent circle diameter of the opening of the well 3 is more preferably 10 $\mu$m or more and 100 $\mu$m or less, and still more preferably 15 $\mu$m or more and 60 $\mu$m or less so that cells can be captured one by one in the well 3.

Here, the beads are particles made of a resin or the like, to which cell components or secretions are adhered for analysis. Beads with components bound to their surfaces for capturing and detecting specific cellular secretions are put into the well 3 together with living cells to identify living cells that secrete a specific secretion or beads with components bound to their surfaces for capturing components constituting cells and cells are put into the well 3, the cells are disrupted, the contents are captured on the beads, the beads are collected outside the well 3, and the contents captured on the beads can be examined. In addition, it is possible to bind a component that releases a drug or the like in response to an external stimulus such as light to the surface of the beads, put the cells and the beads together into the well 3, release the drug by light radiation, and observe the drug response of the cells. In addition, magnetic beads may be used, and the beads may be attracted to a magnet arranged below the well array filter 1 and captured in the well 3.

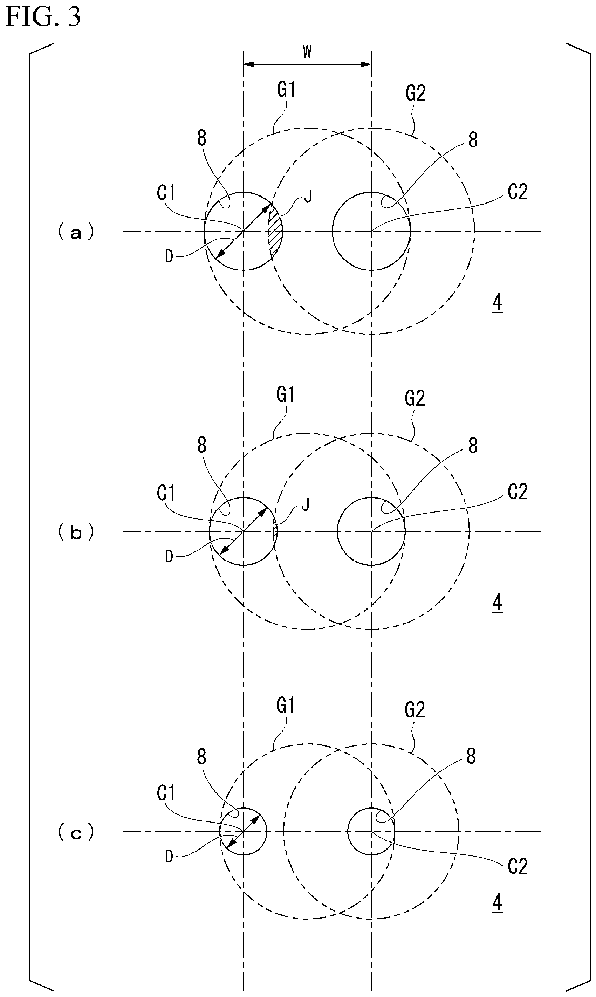

In each well bottom portion 4, a plurality of through-holes 8 that reach the lower surface of the filter body 2 are formed. In this embodiment, the number of through-holes 8 is 2 for each well 3, and as shown in FIG. 3, the through-holes 8 are formed in the center of the well bottom portion 4, with a center-to-center interval W therebetween, to vertically penetrate the well bottom portion 4. In the present invention, the number of through-holes 8 is not limited, and may be three, or four or more, but when a plurality of through-holes 8 are formed in the well bottom portion 4, there is an advantage that cells C are less likely to pass through the through-holes 8 even when a sufficient flow rate is secured, and since at least some through-holes 8 are formed at positions away from the center of the well bottom portion 4, it is rare that all the through-holes 8 are blocked by the cells C, and even after the cells C are captured, it is easy to allow a liquid in the well 3 to circulate appropriately through the through-holes 8.

The minimum width of the upper-surface opening of the through-hole 8 is 1 $\mu$m or more and 3.5 $\mu$m or less. At the same time, the ratio of the total opening area of the through-holes 8 to the area of the well formation region is 0.5% or more and 3.5% or less. Therefore, it is possible to secure the flow rate of the entire well array filter while restricting particles to be captured such as cells or beads from passing through the through-hole.

The minimum width of the upper-surface opening of the through-hole 8 is the width of a part where the width of the opening of the through-hole 8 is narrowest when the through-hole 8 is observed in a plan view, and is, for example, the diameter when the through-hole 8 is a circle, the minor axis when the through-hole 8 is an ellipse, or the length of the short side when the through-hole 8 is a rectangle. The minimum width of the upper-surface opening of the through-hole 8 is more preferably 2 $\mu$m or more and 3.5 $\mu$m or less and still more preferably 2 $\mu$m or more and 2.75 $\mu$m or less.

The total opening area of the through-holes 8 is a total value of the opening areas (=cross-sectional area of the flow path) of all the through-holes 8 formed in the well formation region. When the proportion of the total opening area of the through-holes 8 is 0.50% or more, since the flow resistance of a liquid that passes through the through-holes 8 becomes appropriately small, it is easy to allow the liquid to pass through the well array filter 1 through operation of a pipette. When the proportion of the total opening area of the through-holes 8 is 3.5% or less, a liquid sample does not flow too much when passing through the well array filter 1 through operation of a pipette, and operability is excellent. The proportion of the total opening area of the through-holes 8 is more preferably 0.75% or more and 3.20% or less.

In the present embodiment, as shown in FIGS. 3(a) and 3(b), assuming a minimum circumscribed circle G1 that surrounds all of the through-holes 8 formed in each well bottom portion 4, if the center of the minimum circumscribed circle G1 is moved to either center C1 or C2 of the plurality of through-holes 8, the minimum circumscribed circle G2 after the movement satisfies a positional relationship in which it at least partially overlaps all of the through-holes 8. For example, FIG. 3(a) shows a case in which the center of the minimum circumscribed circle G1 is moved to the center C2 of one of the through-holes 8, and the through-hole 8 with the center C1 on the left side and the minimum circumscribed circle G2 after the movement overlap at an overlapping portion J. Although not shown, if the center of the minimum circumscribed circle G1 is moved to the center C1 of the other through-hole 8, the through-hole 8 with the center C2 on the right side and the minimum circumscribed circle G2 after the movement overlap at an overlapping portion J with the same shape.

The example shown in FIG. 3(b) shows a case in which the distance between the centers C1 and C2 of the through-holes 8 is kept constant, the diameters D of the through-holes 8 are made smaller than in the case of FIG. 3(a), and the center of the minimum circumscribed circle G1 is moved to the center C2 of one of the through-holes 8, and the through-hole 8 with the center C1 and the minimum circumscribed circle G2 after the movement are in contact with each other at the overlapping portion J.

The comparative example shown in FIG. 3(c) shows a case in which the diameter D of the through-hole 8 is made even smaller than in the case of FIG. 3(b), and since the diameter D of the through-hole 8 is small, the through-hole 8 with the center C1 and the minimum circumscribed circle G2 after the movement are separated from each other without overlapping. Therefore, the relationship required for the present invention is not satisfied.

As shown in FIG. 3, when the two through-holes 8 are circles with the same diameter, the above relationship is satisfied when the diameter D of the through-hole 8 and the distance W between the centers of the through-holes 8 satisfy the following formula, and the overlapping portion J is formed.

$$D > W/2$$

As shown in FIGS. 3(a) and 3(b), when the diameter D of the through-hole 8 and the distance W between the centers of the through-holes 8 satisfy the above formula, if one particle such as a cell C is captured in the well 3, there is a high probability of the particle such as a cell C at least partially overlapping all of the plurality of through-holes 8. Therefore, after the first particle such as a cell C is captured, the flow rate of a dispersion medium that passes through all the through-holes 8 in the well 3 is appropriately reduced, the second and subsequent particles such as cells C are restricted from entering the well 3 according to the flow, and there is a high probability of the particle such as a cell C being guided to and captured in an adjacent empty well 3. This increases the probability of particles such as cells C being captured one by one in many wells 3.

The maximum width of the overlapping portion J having the smallest overlapping area among all of the overlapping portions J may be 0.7% or more of the radius of the minimum circumscribed circle G1. The maximum width is a value along a straight line connecting the centers C1 and C2 of the minimum circumscribed circles G1 and G2 before and after the movement. The maximum width may be any of 0.5% or more, 1% or more, 2% or more, 5% or more, 10% or more, 15% or more, 20% or more, and 30% or more of the radius of the minimum circumscribed circle G1. When the shape of the through-hole 8 is a circle as shown in FIG. 3, and the maximum width of the overlapping portion J is 0.7% of the radius of the minimum circumscribed circle G1, the overlapping area of the overlapping portion J having the smallest overlapping area among all of the overlapping portions J corresponds to 0.025% of the opening area of the through-hole 8. Similarly, when the overlapping area of the overlapping portion J is 0.05%, 0.10%, 0.25%, 0.50%, and 1.00% of the opening area of the through-hole 8, the maximum width of the overlapping portion J having the smallest overlapping area corresponds to 1.12%, 1.80%, 3.32%, 5.20%, and 8.26% of the radius of the minimum circumscribed circle G1, respectively. Even if the shape of the through-hole 8 is any shape other than a circle, the overlapping area of the overlapping portion J having the smallest overlapping area among all of the overlapping portions J may be 0.025%, 0.05%, 0.10%, 0.25%, 0.50%, or 1.00% of the opening area of the through-hole 8. The description in this paragraph is incorporated by reference in all of the following embodiments.

On the other hand, in the comparative example shown in FIG. 3(c), since the diameter D of the through-hole 8 and the distance W between the centers of the through-holes 8 do not satisfy Formula (1), and the through-hole 8 with the center C1 and the minimum circumscribed circle G2 after the movement do not overlap each other, when one particle such as a cell C is captured in the well 3, there is a probability of the particle such as a cell C not overlapping all of the through-holes 8. Therefore, even after the first particle such as a cell C is captured, the flow rate of a dispersion medium that passes through the through-holes 8 which do not overlap the particle such as a cell C is still high, and there is a high possibility of the second and subsequent particles such as cells C entering the well 3 according to the flow. This reduces the single cell rate.

The diameter of the minimum circumscribed circle G1 is not limited, and is preferably 3 $\mu$m or more and 30 $\mu$m or less and more preferably 5 $\mu$m or more and 20 $\mu$m or less. The minimum width of the portion separating adjacent through-holes 8 is not limited, and is preferably 0.5 $\mu$m or more and 10 $\mu$m or less, and particularly preferably 1 $\mu$m or more and 3 $\mu$m or less in order to fit the through-hole 8 inside the small minimum circumscribed circle G1.

The maximum thickness B of the well partition wall 6 in the horizontal thickness (refer to FIG. 1) is not limited, and is preferably 1 $\mu$m or more and 20 $\mu$m or less. When the maximum thickness B is 1 $\mu$m or more, the strength of the well partition wall 6 can be made appropriate, and the well partition wall 6 can be prevented from collapsing, for example, when cells C are sucked up by applying a pipette to the upper surface of the well partition wall 6. In addition, when the maximum thickness B is 20 $\mu$m or less, it is possible to restrict particles to be captured such as cells or beads from remaining on the well partition wall 6 and to increase the density of the well 3. In addition, it is possible to impart appropriate flexibility to the well partition wall 6 when a pipette is applied and to restrict damage to the tip of the pipette. The maximum thickness B of the well partition wall 6 is preferably 2 $\mu$m or more and 20 $\mu$m or less and more preferably 2 $\mu$m or more and 12 $\mu$m or less.

The ratio of the total opening area of the wells 3 to the area of the well formation region is not limited, and is preferably 40% or more. When the ratio is 40% or more, the proportion of particles captured in the well 3 among particles such as cells supplied to the well array filter 1, that is, the particle capture rate, can be sufficiently increased. The ratio of the total opening area of the wells 3 to the area of the well formation region is preferably 40% or more and 95% or less and more preferably 40% or more and 75% or less. A high ratio means that the well partition wall 6 is thin.

Although the ratio between the depth of the well 3 and the maximum thickness B of the well partition wall 6 in the horizontal thickness is not limited, the depth of the well 3 is preferably at least twice the thickness B of the well partition wall 6. When the depth of the well 3 is at least twice the thickness B of the well partition wall 6, since it is possible to restrict particles such as cells C captured in the well 3 from escaping from the well 3 due to disturbances in the liquid flow, the particles once captured can be stably held within the well 3. More preferably, the depth of the well 3 may be 2 times to 40 times, and is more preferably 2 times to 15 times, the thickness B of the well partition wall 6.

The shape of the through-hole 8 in a plan view is a circle in this embodiment, but the present invention is not limited to a circle, and the shape may be any shape, such as an ellipse, a polygon such as a triangle, a quadrangle, a pentagon, and a hexagon, a polygon with rounded corners, a rectangle, a star, a slit, a dumbbell shape, an H-shape, and other irregular shapes. Modified examples will be described below.

The shape of the well 3 in a plan view is not limited in the present invention, and may be a circle or ellipse, and when the shape is a polygonal shape or a polygonal shape with rounded corners, it is easy to distinguish the particles from the well 3 during automatic image analysis. Among the polygonal shapes, a triangular shape, a rectangular shape, or a hexagonal shape shown in the drawing is preferable. When the shape is a triangular shape, a rectangular shape, or a hexagonal shape, it is possible to regularly arrange the wells 3 having the same shape and increase the arrangement density of the wells 3. Among these, an equilateral triangular shape, a regular square shape or a regular hexagonal shape is preferable because it is possible to increase the arrangement density of the wells 3 with a geometrically simple arrangement. Modified examples will be described below.

The well partition wall 6 and the well bottom portion 4 may be formed of the same material, but preferably are formed of different materials, and the well bottom portion 4 may be formed of a material having a higher strength than the well partition wall 6. In this case, it is possible to increase the strength of the entire well array filter 1, mitigate shrinkage that occurs during molding of the well bottom portion 4, and reduce warping and distortion of the well array filter 1. It is important that the well array filter 1 be free from warping in order to focus the entire observation area when the particles captured in the well 3 are observed under an inverted microscope.

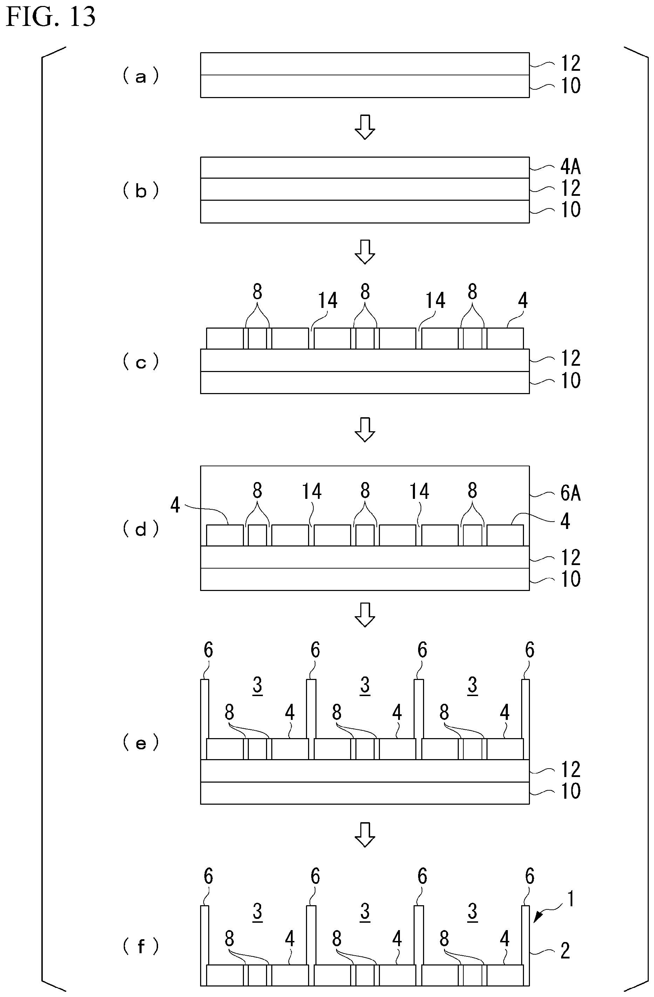

The materials of the well partition wall 6 and the well bottom portion 4 are not limited, but in order to realize a fine structure, various types of photoresists may be used for formation, and the well bottom portion 4 is preferably formed of a material having a higher strength than the well partition wall 6 by changing the type and composition of the photoresist and/or exposure conditions. When the well partition wall 6 and the well bottom portion 4 are formed of a photoresist, for example, a partition wall resist 6A (refer to FIG. 13) forming the well partition wall 6 is formed from a photoresist having a relatively small multifunctional epoxy content, a bottom portion resist 4A (refer to FIG. 13) forming the well bottom portion 4 is formed from a photoresist having a relatively large multifunctional epoxy content, and the strength of the material of the well bottom portion 4 can be made higher than the strength of the material of the well partition wall 6. In this case, the autofluorescence of the material of the well partition wall 6 is relatively weak, the autofluorescence of the material of the well bottom portion 4 is relatively strong, but since the thickness of the well bottom portion 4 is small, it does not adversely affect observation under an inverted microscope.

In the present invention, the method of forming the well bottom portion 4 and the well partition wall 6 is not limited, but in order to reduce stress on the well array filter 1, a structure in which the well partition walls 6 and the well bottom portions 4 are formed separately from each other, the well bottom portions 4 are divided into tiles corresponding to the shapes of the openings of the wells 3, gaps are formed between adjacent well bottom portions 4, and the material forming the well partition wall 6 penetrates into these gaps and solidifies is preferable. An example of a specific producing method will be described below. When the well array filter 1 is formed in such a structure in which the well bottom portions 4 are divided into tiles and the well partition walls 6 penetrate into the gaps in the well bottom portions 4, it is possible to better restrict the well array filter 1 from warping or distorting due to solidification and shrinkage of the well bottom portion 4.

Although not essential in the present invention, at least a part of the inner surface of the well 3 may be coated with a polymer that restricts cell adhesion in advance. In this case, since the polymer applied to the inner surface of the well 3 restricts adhesion of cells C, it is easy to remove the cells C or particles from the well 3. As this type of polymer, for example, 2-methacryloyloxyethyl phosphorylcholine polymers (MPC polymers), polyethylene glycol (PEG), polyvinyl alcohol (PVA), poly(2-methoxyethyl acrylate) (PMEA), and mixtures thereof can be used. The position to which the polymer is applied is not limited to the inner surface of the well 3, but the polymer may be applied to the upper surface of the well partition wall 6, the inner surface of the through-hole 8, and the lower surface of the filter body 2. In this case, it is possible to reduce problems such as adhesion of cells and the like to the upper surface of the well partition wall 6, and adhesion of cells and the like to the lower surface of the filter body 2.

Although not limited in the present invention, the thickness of the well bottom portion 4 may be 1 nm or more and 2 $\mu$m or less. In this case, since the well bottom portion 4 is sufficiently thin, captured particles such as cells C in the well 3 can be easily observed through the well bottom portion 4 under an inverted microscope, and since the well bottom portion 4 is thin, the autofluorescence of the well bottom portion 4 is minimized, and the adverse effect of autofluorescence on observation can also be reduced. The thickness of the well bottom portion 4 is more preferably 100 nm or more and 2 $\mu$m or less, and still more preferably 500 nm or more and 1.5 $\mu$m or less.

According to the well array filter 1 having the above configuration, since the minimum width of the through-hole 8 and the ratio of the total opening area of the through-holes 8 to the area of the well formation region are appropriate, it is possible to restrict particles to be captured such as cells C from passing through the through-hole 8 and secure the flow rate of the entire well array filter 1. In addition, since the plurality of through-holes 8 formed in each well bottom portion 4 satisfy the positional relationship (Formula (1)), if one particle such as a cell C is captured in the well 3, there is a high probability of the particle at least partially overlapping all of the plurality of through-holes 8.

Therefore, as shown in FIG. 2, after the first particle is captured, the flow rate of a dispersion medium that passes through all the through-holes 8 in the well 3 is appropriately reduced, the second and subsequent particles are restricted from entering the same well 3 according to the flow, actually, there is a high probability of cells C and the like being guided to and captured in an adjacent empty well 3. Therefore, there is a high probability of particles being captured one by one in many wells 3, that is, both the capture rate and the single cell rate are high, and the liquid permeability is favorable, and excellent effect of efficient and stable isolation of particles in each well are exhibited.

In addition, in this embodiment, since the well partition wall 6 between the wells 3 is sufficiently thin, particles such as cells or beads are unlikely to remain on the well partition wall 6, the possibility of a plurality of particles entering one well 3 can be reduced by appropriately setting the opening diameter, and particles can be stably captured in the wells 3 that are sufficiently deep compared to the opening diameter. In addition, since the well 3 has a polygonal shape, it is easy to distinguish the particles from the well 3 during automatic image analysis, and furthermore, since the wells 3 can be arranged at a high density, there is an advantage that a large number of particles can be screened at once.

[Well array filter according to second embodiment]

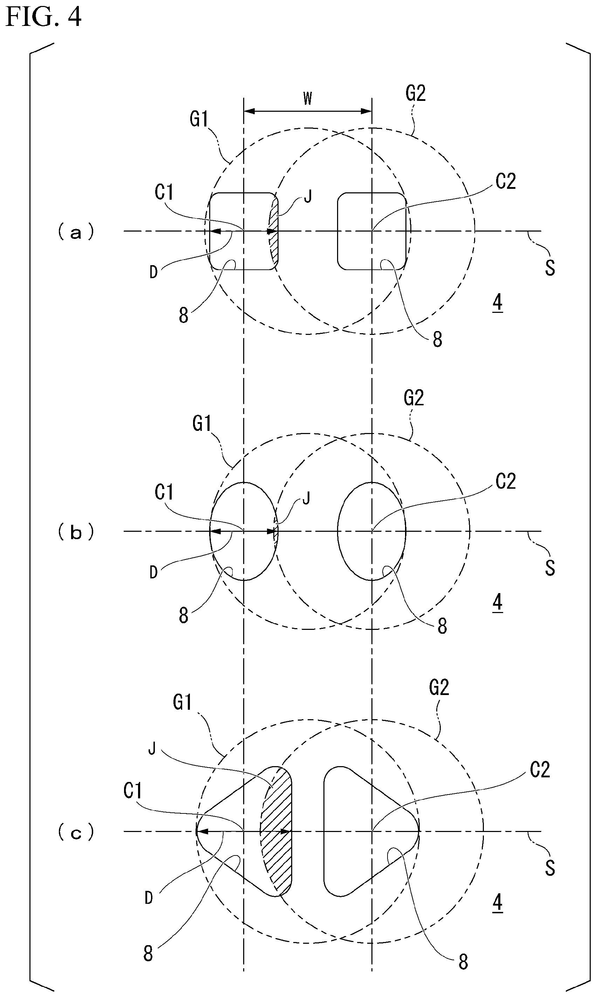

FIG. 4 shows a modified example of the shape of the through-hole 8 when the number of through-holes 8 in each well bottom portion 4 of the well array filter 1 is two.

FIG. 4(a) shows a second embodiment in which the through-holes 8 have a rectangular shape, and are arranged in parallel so that two of their sides are aligned on the same line. The corners of the rectangles are rounded, but the corners need not be rounded. The minimum circumscribed circle G1 passes through two outer vertices of each through-hole 8 and circumscribes each through-hole 8. The centers C1 and C2 of the through-hole 8 are defined as centers of two points where the diameter S of the minimum circumscribed circle G1 extending in a direction in which the through-holes 8 face each other and the opening edge of the through-hole 8 intersect. The same applies to other embodiments.

In the second embodiment as well, assuming a minimum circumscribed circle G1 that surrounds all of the through-holes 8 formed in each well bottom portion 4, if the center of the minimum circumscribed circle G1 is moved to either center C1 or C2 of the through-hole 8, the minimum circumscribed circle G2 after the movement satisfies a positional relationship in which it at least partially overlaps all of the through-holes 8. FIG. 4(a) shows a case in which the center of the minimum circumscribed circle G1 is moved to the center C2 of one of the through-holes 8, and the through-hole 8 with the center C1 on the left side and the minimum circumscribed circle G2 after the movement overlap at an overlapping portion J. Therefore, the same effects as in the first embodiment are obtained, and since the through-hole 8 has a rectangular shape, compared to a circular through-hole, the diameter of the maximum inscribed circle is smaller relative to the opening area, and the possibility of cells C and the like passing through is reduced. Other configurations of the second embodiment may be the same as those of the first embodiment, and the above description is incorporated herein.

[Well array filter according to third embodiment]

FIG. 4(b) shows a third embodiment in which the through-holes 8 have an elliptical shape or an oval shape, and are arranged so that their minor axes (or major axes) are aligned on the same line. The minimum circumscribed circle G1 passes through one point outside each through-hole 8 and circumscribes each through-hole 8. The centers C1 and C2 of the through-hole 8 are centers of two points where the diameter S of the minimum circumscribed circle G1 extending in a direction in which the through-holes 8 face each other and the opening edge of the through-hole 8 intersect.

In the third embodiment as well, assuming a minimum circumscribed circle G1 that surrounds all of the through-holes 8 formed in each well bottom portion 4, if the center of the minimum circumscribed circle G1 is moved to either center C1 or C2 of the through-hole 8, the minimum circumscribed circle G2 after the movement satisfies a positional relationship in which it at least partially overlaps all of the through-holes 8. FIG. 4(b) shows a case in which the center of the minimum circumscribed circle G1 is moved to the center C2 of one of the through-holes 8 and the through-hole 8 with the center C1 on the left side and the minimum circumscribed circle G2 after the movement overlap at an overlapping portion J. Therefore, the same effects as in the first embodiment are obtained, and since the through-hole 8 has an elliptical shape, compared to a circular through-hole, the diameter of the maximum inscribed circle is smaller relative to the opening area, and the possibility of cells C and the like passing through is reduced. Other configurations of the third embodiment may be the same as those of the first embodiment, and the above description is incorporated herein.

[Well array filter according to fourth embodiment]

FIG. 4(c) shows a fourth embodiment in which the through-holes 8 have a triangular shape, preferably, an isosceles triangular shape, and are arranged with their bases facing each other in parallel. The corners of the triangles are rounded, but the corners need not be rounded. The minimum circumscribed circle G1 passes through outer vertexes of each through-hole 8 and circumscribes each through-hole 8. The centers C1 and C2 of the through-hole 8 are centers of two points where the diameter S of the minimum circumscribed circle G1 extending in a direction in which the through-holes 8 face each other and the opening edge of the through-hole 8 intersect.

In the fourth embodiment as well, assuming a minimum circumscribed circle G1 that surrounds all of the through-holes 8 formed in each well bottom portion 4, if the center of the minimum circumscribed circle G1 is moved to either center C1 or C2 of the through-hole 8, the minimum circumscribed circle G2 after the movement satisfies a positional relationship in which it at least partially overlaps all of the through-holes 8. FIG. 4(c) shows a case in which the center of the minimum circumscribed circle G1 is moved to the center C2 of one of the through-holes 8, and the through-hole 8 with the center C1 on the left side and the minimum circumscribed circle G2 after the movement overlap at an overlapping portion J. Therefore, the same effects as in the first embodiment are obtained, and since the through-hole 8 has a triangular shape, compared to a circular through-hole, the diameter of the maximum inscribed circle is smaller relative to the opening area, and the possibility of cells C and the like passing through is reduced. In addition, since the bases of the triangles are arranged facing each other, it is easy to increase the area of the overlapping portion J, and it is possible to increase the flow path blockage rate when cells C and the like are placed. Other configurations of the fourth embodiment may be the same as those of the first embodiment, and the above description is incorporated herein.

[Well array filter according to fifth embodiment]

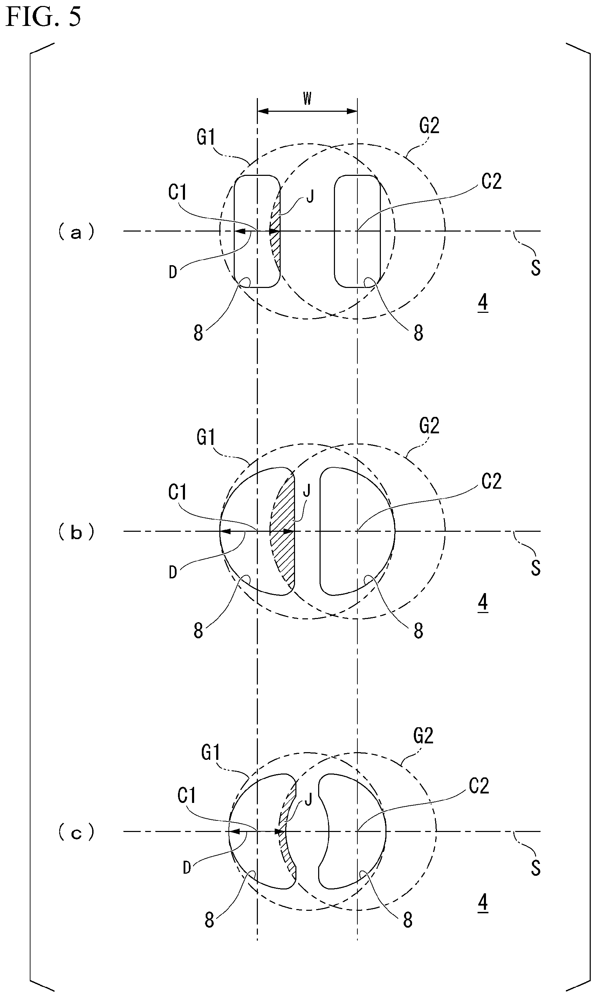

FIG. 5 shows a modified example in which the shape of the through-hole 8 is elongated when the number of through-holes 8 in each well bottom portion 4 of the well array filter 1 is two.

FIG. 5(a) shows a fifth embodiment in which the through-holes 8 have an elongated rectangular shape, and are arranged in parallel so that their two short sides are aligned on the same line. The corners of the rectangles are rounded, but the corners need not be rounded. The minimum circumscribed circle G1 passes through two outer vertices of each through-hole 8 and circumscribes each through-hole 8. The centers C1 and C2 of the through-hole 8 are centers of two points where the diameter S of the minimum circumscribed circle G1 extending in a direction in which the through-holes 8 face each other and the opening edge of the through-hole 8 intersect.

In the fifth embodiment as well, assuming a minimum circumscribed circle G1 that surrounds all of the through-holes 8 formed in each well bottom portion 4, if the center of the minimum circumscribed circle G1 is moved to either center C1 or C2 of the through-hole 8, the minimum circumscribed circle G2 after the movement satisfies a positional relationship in which it at least partially overlaps all of the through-holes 8. FIG. 5(a) shows a case in which the center of the minimum circumscribed circle G1 is moved to the center C2 of one of the through-holes 8, and the through-hole 8 with the center C1 on the left side and the minimum circumscribed circle G2 after the movement overlap at an overlapping portion J. Therefore, the same effects as in the first embodiment are obtained, and since the through-hole 8 has an elongated rectangular shape, compared to a circular through-hole, the diameter of the maximum inscribed circle is smaller relative to the opening area, and the possibility of cells C and the like passing through is reduced. In addition, since the long sides of the rectangular shapes are arranged facing each other, it is easy to increase the area of the overlapping portion J, and it is possible to increase the flow path blockage rate when cells C and the like are placed. Other configurations of the fifth embodiment may be the same as those of the first embodiment, and the above description is incorporated herein.

[Well array filter according to sixth embodiment]

FIG. 5(b) shows a sixth embodiment in which the through-holes 8 have a semicircular shape, and are arranged with their chords facing each other in parallel. The corners of the semicircles are rounded, but the corners need not be rounded. The minimum circumscribed circle G1 passes through outer vertices of each through-hole 8 and circumscribes each through-hole 8. The centers C1 and C2 of the through-hole 8 are centers of two points where the diameter S of the minimum circumscribed circle G1 extending in a direction in which the through-holes 8 face each other and the opening edge of the through-hole 8 intersect.

In the sixth embodiment as well, assuming a minimum circumscribed circle G1 that surrounds all of the through-holes 8 formed in each well bottom portion 4, if the center of the minimum circumscribed circle G1 is moved to either center C1 or C2 of the through-hole 8, the minimum circumscribed circle G2 after the movement satisfies a positional relationship in which it at least partially overlaps all of the through-holes 8. FIG. 5(b) shows a case in which the center of the minimum circumscribed circle G1 is moved to the center C2 of one of the through-holes 8, and the through-hole 8 with the center C1 on the left side and the minimum circumscribed circle G2 after the movement overlap at an overlapping portion J. Therefore, the same effects as in the first embodiment are obtained, and since the through-hole 8 has a semicircular shape, compared to a circular through-hole, the diameter of the maximum inscribed circle is smaller relative to the opening area, and the possibility of cells C and the like passing through is reduced. In addition, since the chords of the semicircles are arranged facing each other, it is easy to increase the area of the overlapping portion J, and it is possible to increase the flow path blockage rate when cells C and the like are placed. Other configurations of the sixth embodiment may be the same as those of the first embodiment, and the above description is incorporated herein.

[Well array filter according to seventh embodiment]

FIG. 5(c) shows a seventh embodiment in which the through-holes 8 have a semicircular shape and are arranged with their chords facing each other in parallel, and the chord is formed in an arc shape to form a so-called banana shape. The corners of the semicircles are rounded, but the corners need not be rounded. The minimum circumscribed circle G1 passes through outer vertices of each through-hole 8 and circumscribes each through-hole 8. The centers C1 and C2 of the through-hole 8 are centers of two points where the diameter S of the minimum circumscribed circle G1 extending in a direction in which the through-holes 8 face each other and the opening edge of the through-hole 8 intersect.

In the seventh embodiment as well, assuming a minimum circumscribed circle G1 that surrounds all of the through-holes 8 formed in each well bottom portion 4, if the center of the minimum circumscribed circle G1 is moved to either center C1 or C2 of the through-hole 8, the minimum circumscribed circle G2 after the movement satisfies a positional relationship in which it at least partially overlaps all of the through-holes 8. FIG. 5(c) shows a case in which the center of the minimum circumscribed circle G1 is moved to the center C2 of one of the through-holes 8, and the through-hole 8 with the center C1 on the left side and the minimum circumscribed circle G2 after the movement overlap at an overlapping portion J. Therefore, the same effects as in the first embodiment are obtained, and since the through-hole 8 has a banana shape, compared to a circular through-hole, the diameter of the maximum inscribed circle is smaller relative to the opening area, and the possibility of cells C and the like passing through is reduced. In addition, since the concave sides of the banana shapes are arranged facing each other, it is easy to increase the area of the overlapping portion J, it is possible to increase the flow path blockage rate when cells C and the like are placed, and it is also possible to increase the area on which cells C can be placed. Other configurations of the seventh embodiment may be the same as those of the first embodiment, and the above description is incorporated herein.

[Well array filter according to eighth embodiment]

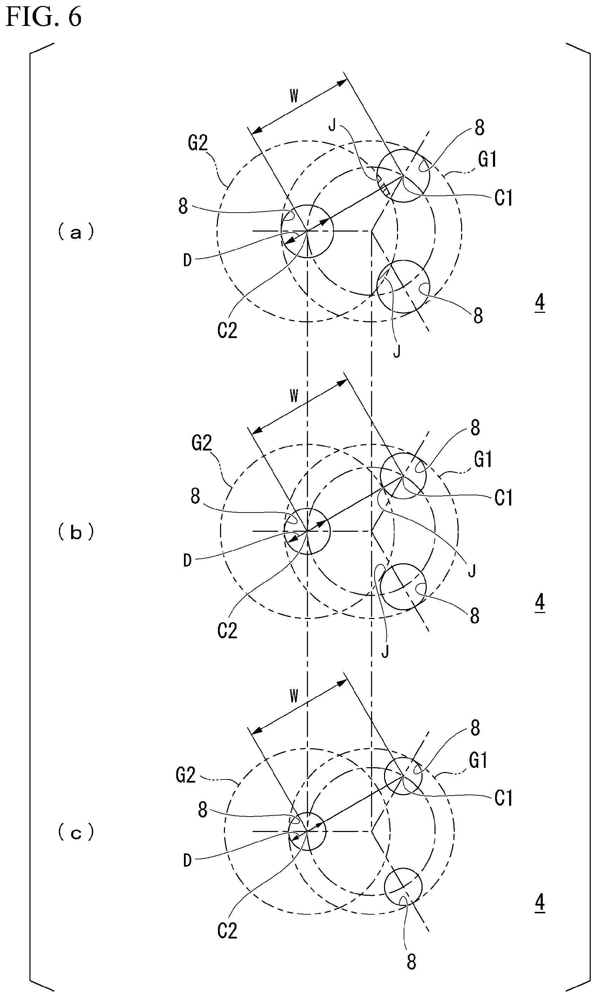

FIG. 6 shows a case in which a total of three circular through-holes 8 are formed in the center of the well bottom portion 4.

FIG. 6(a) shows an eighth embodiment, and in this example, a total of three circular through-holes 8 are formed in the center of the well bottom portion 4. The through-holes 8 may be formed at the vertex positions of an equilateral triangle, but may also be formed at the vertices of an isosceles triangle, a right triangle, or a scalene triangle rather than an equilateral triangle. When arranged in a triangular shape, it is preferable that the center of the well bottom portion 4 be positioned inside the triangle, preferably, at the center of gravity. Other configurations of the eighth embodiment may be the same as those of the first embodiment, and the above description is incorporated herein.

In the eighth embodiment as well, assuming a minimum circumscribed circle G1 that surrounds all of the through-holes 8 formed in each well bottom portion 4, if the center of the minimum circumscribed circle G1 is moved to the center of any of the three through-holes 8, the minimum circumscribed circle G2 after the movement satisfies a positional relationship in which it at least partially overlaps all of the through-holes 8. FIG. 6(a) shows a case in which the center of the minimum circumscribed circle G1 is moved to the center C2 of one of the through-holes 8, and two through-holes 8 with the center C1 on the right side and the minimum circumscribed circle G2 after the movement overlap at an overlapping portion J. Therefore, the same effects as in the first embodiment are obtained. In addition, there is an advantage that a relatively large area for placing cells C can be secured in the center of the three through-holes 8.

When the diameter D of the through-hole 8 in the eighth embodiment is reduced, as shown in FIG. 6(b), when the center of the minimum circumscribed circle G1 is moved to the center C2 of one of the through-holes 8, the two through-holes 8 with the center C1 on the right side and the minimum circumscribed circle G2 after the movement come into contact with each other in the overlapping portion J, and when the diameter is further reduced, as shown in FIG. 6(c), when the center of the minimum circumscribed circle G1 is moved to the center C2 of one of the through-holes 8, the two through-holes 8 with the center C1 on the right side and the minimum circumscribed circle G2 after the movement do not overlap. Therefore, FIG. 6(c) shows a comparative example.

[Well array filter according to ninth embodiment]

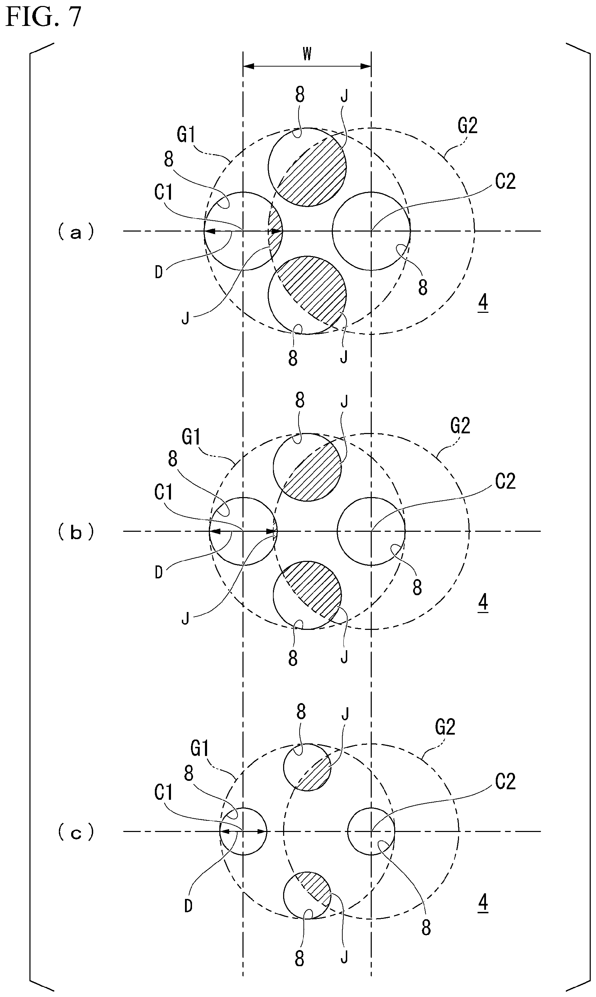

FIG. 7 shows a case in which a total of four through-holes 8 are formed in the center of the well bottom portion 4.

FIG. 7(a) shows a ninth embodiment in which a total of four through-holes 8 are formed in the center of the well bottom portion 4. The through-holes 8 may be formed at the vertex positions of a square, but may also be formed at the vertices of a rectangle, a trapezoid, or a scalene quadrangle other than a square. When arranged in a quadrangular shape, it is preferable that the center of the well bottom portion 4 be positioned inside the quadrangle, preferably, at the center of gravity. Alternatively, three through-holes 8 may be arranged at the vertex positions of a triangle, and a fourth through-hole 8 may be arranged inside the triangle or on one of the three sides. In this case, the triangle may be an equilateral triangle, but may also be an isosceles triangle, a right triangle, or a scalene triangle rather than an equilateral triangle. When arranged in a triangular shape, it is preferable that the center of the well bottom portion 4 be positioned inside the triangle, preferably, at the center of gravity. Other configurations of the ninth embodiment may be the same as those of the first embodiment, and the above description is incorporated herein.

In the ninth embodiment as well, assuming a minimum circumscribed circle G1 that surrounds all of the through-holes 8 formed in each well bottom portion 4, if the center of the minimum circumscribed circle G1 is moved to the center of any of the four through-holes 8, the minimum circumscribed circle G2 after the movement satisfies a positional relationship in which it at least partially overlaps all of the through-holes 8. FIG. 7(a) shows a case in which the center of the minimum circumscribed circle G1 is moved to the center C2 of the through-hole 8 on the right, and the three through-holes 8 on the left side and the minimum circumscribed circle G2 after the movement overlap in the overlapping portion J. Therefore, the same effects as in the first embodiment are obtained. In addition, there is an advantage that a relatively large area for placing cells C can be secured in the center of the three through-holes 8.

When the diameter D of the through-hole 8 in the ninth embodiment is reduced, as shown in FIG. 7(b), when the center of the minimum circumscribed circle G1 is moved to the center C2 of one of the through-holes 8, the two through-holes 8 with the center C1 on the right side and the minimum circumscribed circle G2 after the movement come into contact with each other in the overlapping portion J. When the diameter is further reduced, as shown in FIG. 7(c), when the center of the minimum circumscribed circle G1 is moved to the center C2 of one of the through-holes 8, the through-hole 8 with the center C1 on the left side and the minimum circumscribed circle G2 after the movement do not overlap. Therefore, FIG. 7(c) is a comparative example.

[Other comparative examples of well array filter]

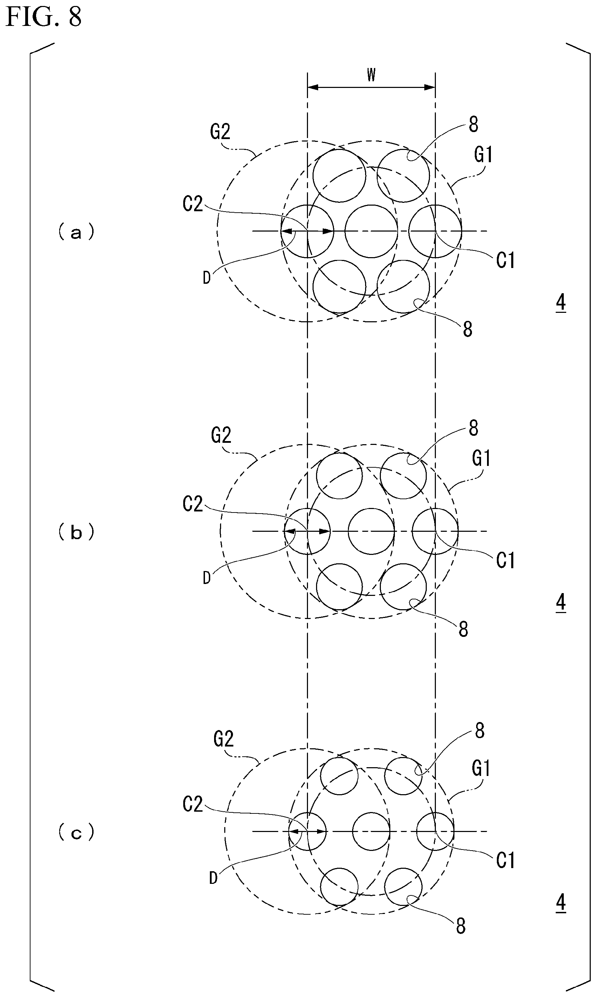

FIG. 8 shows an example in which a total of seven through-holes 8 are formed in the center of the well bottom portion 4.

In all of FIGS. 8(a) to 8(c), six through-holes 8 are formed at the vertex positions of a regular hexagon, and a seventh through-hole 8 is formed in the center of the regular hexagon. With this arrangement, as long as the opening diameters of the through-holes 8 are the same, as shown in FIG. 8, when the center of the minimum circumscribed circle G1 is moved to the center C2 of one of the through-holes 8, the through-hole 8 with the center C1 on the left side and the minimum circumscribed circle G2 after the movement cannot overlap. Therefore, FIGS. 8(a) to 8(c) are all comparative examples.

[Well array filter according to tenth embodiment]

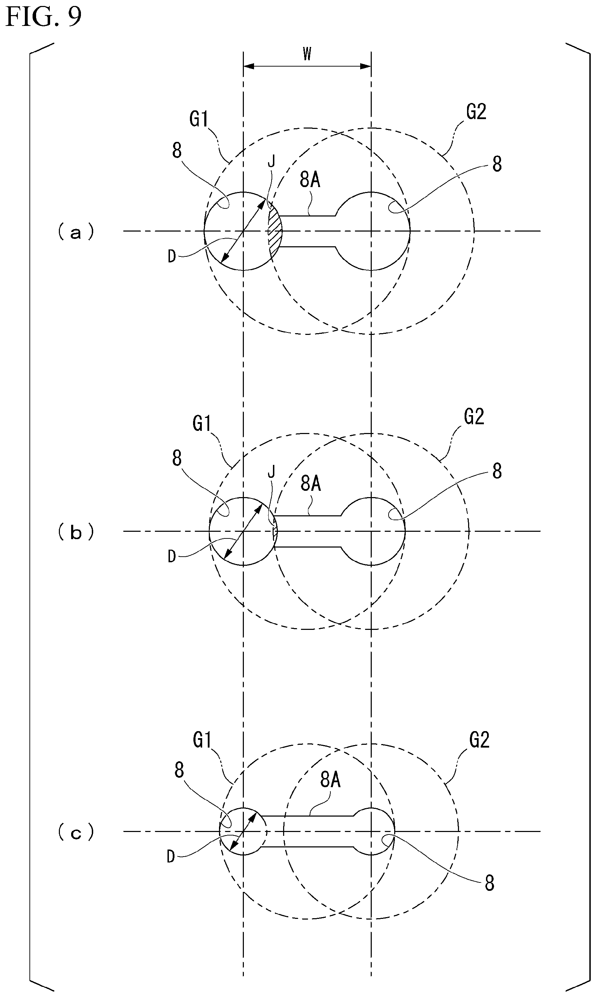

FIG. 9 shows a well array filter 1 according to a tenth embodiment, and in this example, in the center of the well bottom portion 4, a through-hole 8 having a planar shape (dumbbell shape) is formed by connecting two circular through-holes 8 with a slit 8A. In the case of the through-hole 8 having such a shape, even when the maximum inscribed circle diameter of the through-hole 8 is 4 $\mu$m or less, the opening area (cross-sectional area of the flow path) of the through-hole 8 as large as the slit 8A can be secured, and the flow rate of the through-hole 8A can be increased while preventing particles such as cells or beads from passing through the through-hole 8A. In this case, it is preferable that the center of the well bottom portion 4 be positioned on the through-hole 8A. Other configurations of the tenth embodiment may be the same as those of the first embodiment, and the above description is incorporated herein.

In the tenth embodiment as well, assuming a minimum circumscribed circle G1 that surrounds all of the through-holes 8 formed in each well bottom portion 4, if the center of the minimum circumscribed circle G1 is moved to the center of any of the through-holes 8, the minimum circumscribed circle G2 after the movement satisfies a positional relationship in which it at least partially overlaps all of the through-holes 8. FIG. 9(a) shows a case in which the center of the minimum circumscribed circle G1 is moved to the center C2 of the through-hole 8 on the right side, and the through-hole 8 on the left side and the minimum circumscribed circle G2 after the movement overlap at an overlapping portion J. Therefore, the same effects as in the first embodiment are obtained.

When the diameter D of the through-hole 8 in the tenth embodiment is reduced, as shown in FIG. 9(b), when the center of the minimum circumscribed circle G1 is moved to the center C2 of one of the through-holes 8, the two through-holes 8 with the center C1 on the right side and the minimum circumscribed circle G2 after the movement come into contact with each other in the overlapping portion J. When the diameter is further reduced, as shown in FIG. 9(c), when the center of the minimum circumscribed circle G1 is moved to the center C2 of one of the through-holes 8, the through-hole 8 with the center C1 on the left side and the minimum circumscribed circle G2 after the movement do not overlap. Therefore, FIG. 9(c) is a comparative example.

[Well array filter according to eleventh embodiment]

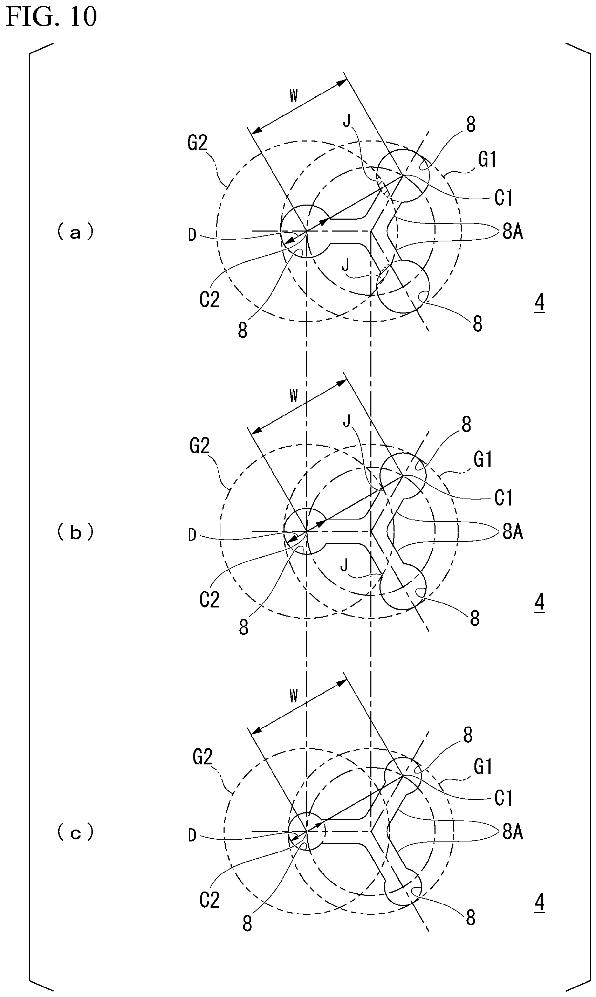

FIGS. 10(a) and 10(b) show a well array filter 1 according to an eleventh embodiment, and in this example, in the center of the well bottom portion 4, three slits 8A extending radially from the center, and through-holes 8 formed at the ends of the slits 8A are formed. In the case of the through-holes 8 connected by such slits 8A, even if the maximum inscribed circle diameter is small, the opening area (cross-sectional area of the flow path) as large as the slit 8A can be secured. In this case, it is preferable that the center of the well bottom portion 4 be positioned at the center of the radial slit 8A. Other configurations of the eleventh embodiment may be the same as those of the first embodiment, and the above description is incorporated herein.

In the eleventh embodiment as well, assuming a minimum circumscribed circle G1 that surrounds all of the through-holes 8 formed in each well bottom portion 4, if the center of the minimum circumscribed circle G1 is moved to the center of any of the through-holes 8, the minimum circumscribed circle G2 after the movement satisfies a positional relationship in which it at least partially overlaps all of the through-holes 8. FIGS. 10(a) and 10(b) show cases in which the center of the minimum circumscribed circle G1 is moved to the center C2 of the through-hole 8 on the right side, and the through-hole 8 on the left side and the minimum circumscribed circle G2 after the movement overlap at an overlapping portion J. Therefore, the same effects as in the first embodiment are obtained.

When the diameter D of the through-hole 8 in the eleventh embodiment is further reduced, as shown in FIG. 10(c), when the center of the minimum circumscribed circle G1 is moved to the center C2 of one of the through-holes 8, the through-hole 8 with the center C1 on the left side and the minimum circumscribed circle G2 after the movement do not overlap. Therefore, FIG. 10(c) is a comparative example.

[Well array filter according to twelfth embodiment]

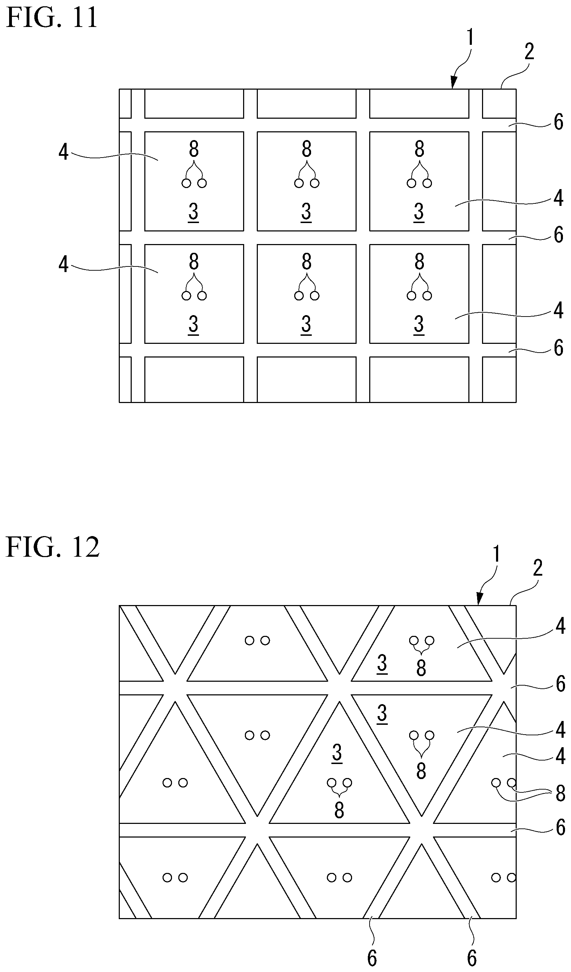

FIG. 11 shows a well array filter 1 according to a twelfth embodiment, and in this example, the well partition wall 6 is formed in a square grid shape, and the well bottom portions 4 are each formed in a square shape. The well partition wall 6 is not limited to a square grid shape, and may have an oblique grid shape or a rectangular grid shape, but it is preferable that the shapes of the individual wells 3 be the same. The wells 3 may be arranged alternately in a zigzag manner in every other row. The through-hole 8 may be the same as in any of the first to eleventh embodiments. Other configuration of the twelfth embodiment may be the same as those of the first embodiment, and the above description is incorporated herein. In the twelfth embodiment as well, the same effects as in the first to eleventh embodiments are obtained.

[Well array filter according to thirteenth embodiment]

FIG. 12 shows a well array filter 1 according to a thirteenth embodiment, and in this example, the well partition walls 6 are formed in a so-called truss shape, with equilateral triangles arranged alternately in opposite directions, and the well bottom portions 4 are each formed in an equilateral triangular shape. The well partition wall 6 is not limited to an equilateral triangular shape, and may have an isosceles triangular shape, a right triangle shape, or a scalene triangular shape, but it is preferable that the shapes of the individual wells 3 be the same. The through-hole 8 may be the same as in any of the first to eleventh embodiments. Other configurations of the thirteenth embodiment may be the same as those of the first embodiment, and the above description is incorporated herein. In the thirteenth embodiment as well, the same effects as in the first to twelfth embodiments are obtained.

[Method of producing well array filter according to one embodiment]

FIGS. 13(a) to 13(f) are cross-sectional views illustrating an example of a method of producing a well array filter 1. First, as shown in FIG. 13(a), on a substrate 10 made of a Si wafer or the like, a sacrificial film 12 that is insoluble in a resist solvent and a developing solution is formed. The sacrificial film 12 can be formed of, for example, a styrene-based elastomer, dextrin, or dextran.

Next, as shown in FIG. 13(b), on the sacrificial film 12, a negative bottom portion resist 4A is formed to a certain thickness required for the well bottom portion 4. The bottom portion resist 4A is exposed to a predetermined pattern by optical lithography and additionally developed, and as shown in FIG. 13(c), the well bottom portions 4 having the through-holes 8 are arranged in the form of polygonal tiles with gaps 14 therebetween. The width of the gap 14 is set to be slightly smaller than the thickness of the well partition wall 6. In addition, the gaps 14 between the well bottom portions 4 may be partially crosslinked by thin bridges. In this case, positioning can be made so that the arrangement of the well bottom portions 4 is not disturbed by the bridges.

Next, as shown in FIG. 13(d), the partition wall resist 6A covering the well bottom portion 4 is formed to a thickness required for the well partition wall 6. In this case, for example, it is preferable to form the partition wall resist 6A from a photoresist having a relatively small multifunctional epoxy content and the bottom portion resist 4A from a photoresist having a relatively large multifunctional epoxy content so that the strength of the material of the well bottom portion 4 is relatively higher than the strength of the material of the well partition wall 6. In this case, as a secondary effect, the autofluorescence of the material of the well partition wall 6 is relatively weak, the autofluorescence of the material of the well bottom portion 4 is relatively strong, but since the thickness of the well bottom portion 4 is small, it does not adversely affect observation under an inverted microscope. The partition wall resist 6A is exposed to a predetermined pattern by optical lithography and additionally developed, and as shown in FIG. 13(e), the well partition wall 6 is formed. Therefore, the partition wall resist 6A penetrates into the gaps 14 and is insolubilized in the gaps 14 by exposure, and the well bottom portions 4 are connected to each other.

Next, the well bottom portions 4 and the well partition walls 6 are all heated to, for example, 180°C, the resist is additionally thermally cured, the sacrificial film 12 is then dissolved in a solvent that does not alter the resist, the substrate 10 is peeled off of the well bottom portion 4, and the well array filter 1 as shown in the first to thirteenth embodiments is completed.

According to the production method, the well array filter 1 having wells 3 with high shape accuracy can be efficiently produced. In addition, according to this production method, after the well bottom portions 4 are divided into tiles to form the gaps 14 therebetween, the well partition wall 6 is formed and the material forming the well partition wall 6 penetrates into the gaps 14, and thus it is possible to reduce warping and distortion occurring in the well array filter 1 due to solidification and shrinkage of the well bottom portion 4.

[Particle alignment device according to embodiment]

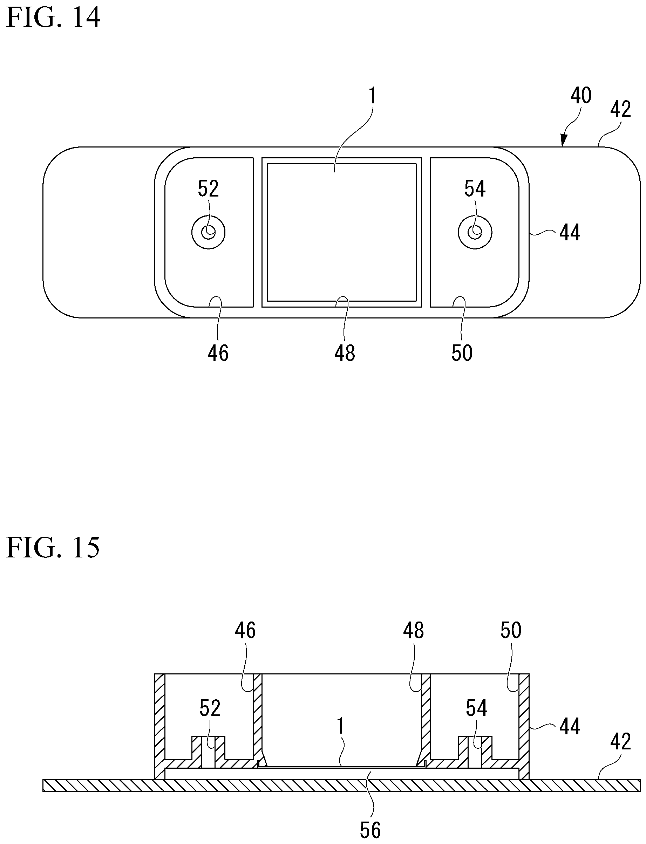

FIG. 14 and FIG. 15 are a plan view and a longitudinal cross-sectional view showing a particle alignment device 40 according to one embodiment of the present invention. The particle alignment device 40 has a rectangular bottom plate portion 42 and a wall portion 44 formed in the center of the bottom plate portion 42. The wall portion 44 has rectangular frame-shaped peripheral wall portion that rises from the bottom plate portion 42 and two partition walls that rise from the inside of the peripheral wall portion and are formed in parallel to each other, and the inside of the peripheral wall portion is divided into a first chamber 46, a second chamber 48, and a third chamber 50 by the partition walls. A cylindrical opening 52 is formed in the bottom portion of the first chamber 46, and a cylindrical opening 54 is formed in the bottom portion of the third chamber 50.

The bottom of the second chamber 48 is open, and as shown in FIG. 15, the well array filter 1 is fixed to the lower end of the wall portion 44 with a small flow path 56 spaced from the bottom plate portion 42. A certain gap is provided between the bottom portion of the first chamber 46 and the bottom plate portion 42, and between the bottom portion of the third chamber 50 and the bottom plate portion 42 to form the flow path 56. Therefore, a liquid sample containing particles such as cells C or beads enters the second chamber 48, the liquid flows out from the opening 52 or the opening 54, and thus particles such as cells C or beads can be captured in the wells 3 of the well array filter 1. That is, a sample flow path that reaches the flow path 56 through the second chamber 48, the well array filter 1, and the through-hole 8 is formed. In addition, a liquid reagent can be introduced from the opening 52 or the opening 54 to react with particles such as cells C or beads in the second chamber 48.

[Particle capture method according to embodiment]

A particle capture method according to one embodiment of the present invention includes a step of passing a liquid containing an organic solvent that does not dissolve or alter the well array filter 1 and the device body 40 through a sample flow path through the well array filter 1 of the particle alignment device 40, that is, a sample flow path from the side of the opening of the well 3 to the bottom side of the well 3 through the through-hole 8, a step of passing an aqueous solution through the sample flow path and replacing the liquid containing the organic solvent with the aqueous solution, and a step of passing an aqueous solution containing particles to be captured in the well 3 through the sample flow path and capturing the particles in the well 3.

Specifically, a liquid such as a water-soluble and low-interfacial-tension organic solvent such as ethyl alcohol, or an aqueous solution containing about 30 to 80% of an organic solvent such as ethyl alcohol is supplied to the second chamber 48 of the particle alignment device 40, and the liquid containing the organic solvent is passed through the well array filter 1. As the liquid, for example, an aqueous solution containing 70% of ethyl alcohol can be suitably used. Therefore, the through-hole 8 is also filled with the organic solvent. Next, an aqueous solution of a buffer such as a phosphate buffer (PBS) is added to the second chamber 48, and the liquid containing the organic solvent is aspirated from the opening 52 and/or the opening 54. This operation is repeated several times to replace the liquid containing the organic solvent inside the second chamber 48, the through-hole 8, and the flow path 56 with an aqueous phosphate buffer solution.

Next, a sample liquid in which particles such as cells C and/or beads are dispersed in a phosphate buffer or the like is supplied to the second chamber 48, the phosphate buffer is aspirated from the opening 52 and/or the opening 54, and thus the cells C and/or beads are captured by one by one or close to certain numbers in each well 3.

Next, a reagent that binds to target cells C or beads to which cell secretions are adhered is added through the second chamber 48 or the openings 52 and 54, the target is marked, the target is then identified under an inverted microscope, and the target cells C or beads are collected from the well 3 using a pipette, a capillary or the like.

According to the above particle capture method, when the liquid containing the organic solvent is passed through the sample flow path through the well array filter 1, and additionally replaced with the aqueous solution, and the aqueous solution containing particles such as cells, beads to which cell components or secretions are adhered, or beads for adhering cell components or secretions is then passed through, the liquid passage resistance is reduced, air bubbles are less likely to remain in the wells 3, and it becomes easy to captures particles such as cells C or beads one by one or in predetermined numbers in each well 3. Therefore, in combination with the effects of the well array filter 1 according to the first embodiment to the thirteenth embodiment, there is an advantage that a large number of particles can be screened efficiently and stably.

While the embodiments of the present invention have been described above, the present invention is not limited to these embodiments, and additions, deletions and modifications of constituent elements can be made with the scope of the claims.

EXAMPLES

Next, examples of the present invention will be described.

[Experiment 1]

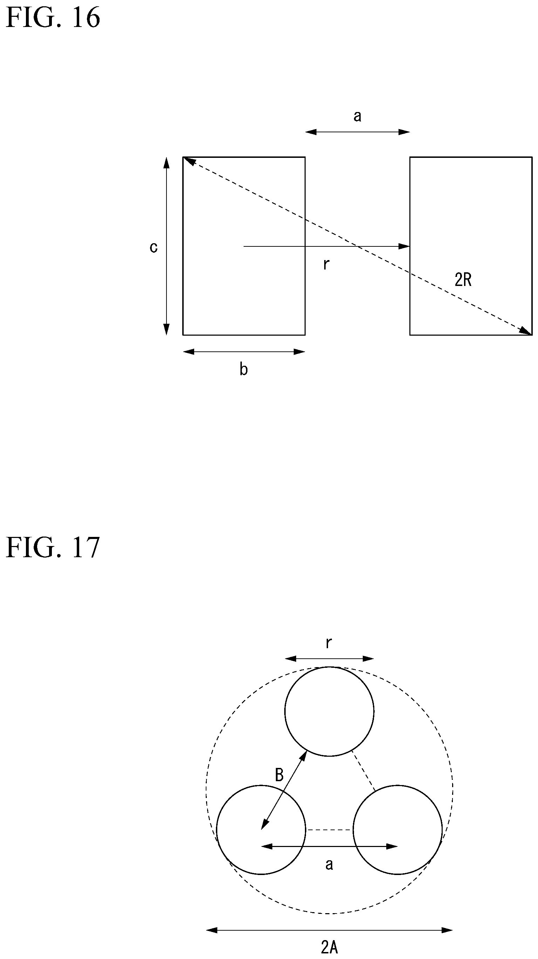

As shown in FIG. 16, conditions for realizing examples of the present invention were determined when two identical rectangular through-holes 8 were arranged so that two of their sides were aligned on straight lines. a ($\mu$m) is the separation distance between the through-holes 8, b ($\mu$m) is the length of the horizontal side of the rectangular through-hole 8, c ($\mu$m) is the length of the vertical side, R ($\mu$m) is 1/2 of the distance from the upper left vertex of the through-hole 8 on the left side to the lower right vertex of the through-hole 8 on the right side, and r ($\mu$m) is the length from the center of one through-hole 8 to the other through-hole 8. The distance R ($\mu$m) is the radius of the minimum circumscribed circle. The length r ($\mu$m) is the minimum contact radius of a circle with the center of the minimum circumscribed circle that is moved to the center of the through-hole 8 on the left side and with the circumference at which the minimum circumscribed circle after the movement is in contact with the through-hole 8 on the right side.

The radius R ($\mu$m) of the minimum circumscribed circle is calculated using the separation distance a ($\mu$m), the length b ($\mu$m) of the horizontal side, and the length c ($\mu$m) of the vertical side as follows.

[Math. 1]

$$R = \frac{1}{2}\sqrt{(a + 2b)^2 + c^2}$$

The minimum contact radius r ($\mu$m) is calculated using the separation distance a ($\mu$m), and the length b ($\mu$m) of the horizontal side as follows.

[Math. 2]

$$r = a + \frac{b}{2}$$

Therefore, if the separation distance between the through-holes 8 is set to a ($\mu$m), the length of the horizontal side of the rectangular through-hole 8 is set to b ($\mu$m), and the length of the vertical side is set to c ($\mu$m) so that R>r is satisfied, conditions for examples of the present invention are satisfied. Specific examples of cases in which conditions for examples are satisfied (examples), and cases in which conditions are not satisfied (comparative examples) are shown below.

[Example in which conditions are satisfied]

The separation distance a between the through-holes 8: 1.00 ($\mu$m), the length b of the horizontal side of the rectangular through-hole 8: 2.50 ($\mu$m), the length c of the vertical side: 6.02 ($\mu$m), the diameter 2R of the minimum circumscribed circle: 8.50 ($\mu$m) [Example in which conditions are not satisfied]

The separation distance a between the through-holes 8: 3.00 ($\mu$m), the length b of the horizontal side of the rectangular through-hole 8: 2.50 ($\mu$m), the length c of the vertical side: 2.87 ($\mu$m), the diameter 2R of the minimum circumscribed circle: 8.50 ($\mu$m)

[Experiment 2]

As shown in FIG. 17, conditions for realizing examples of the present invention were determined when three identical through-holes 8 were arranged so that the centers of the circular through-holes 8 were located at the vertex positions of an equilateral triangle. a ($\mu$m) is the separation distance between the centers of the through-holes 8, r ($\mu$m) is the diameter of the through-hole 8, A ($\mu$m) is the radius of the minimum circumscribed circle, and B ($\mu$m) is the length from the center of one through-hole 8 to the other through-hole 8. The length B ($\mu$m) is the minimum contact radius of a circle with the center of the minimum circumscribed circle that is moved to the center of the lower left through-hole 8 and with the circumference at which the minimum circumscribed circle after the movement is in contact with the through-hole 8 on the upper side.

The radius A ($\mu$m) of the minimum circumscribed circle is represented by the following formula.

[Math. 3]

$$A = \frac{1}{\sqrt{3}} a + \frac{1}{2} r$$

The minimum contact radius B ($\mu$m) is represented by the following formula.

[Math. 4]

$$B = a - \frac{1}{2} r$$

Therefore, the width of the overlapping portion J is represented by the following formula.

[Math. 5]

$$A - B = r - \left(1 - \frac{1}{\sqrt{3}}\right) a$$

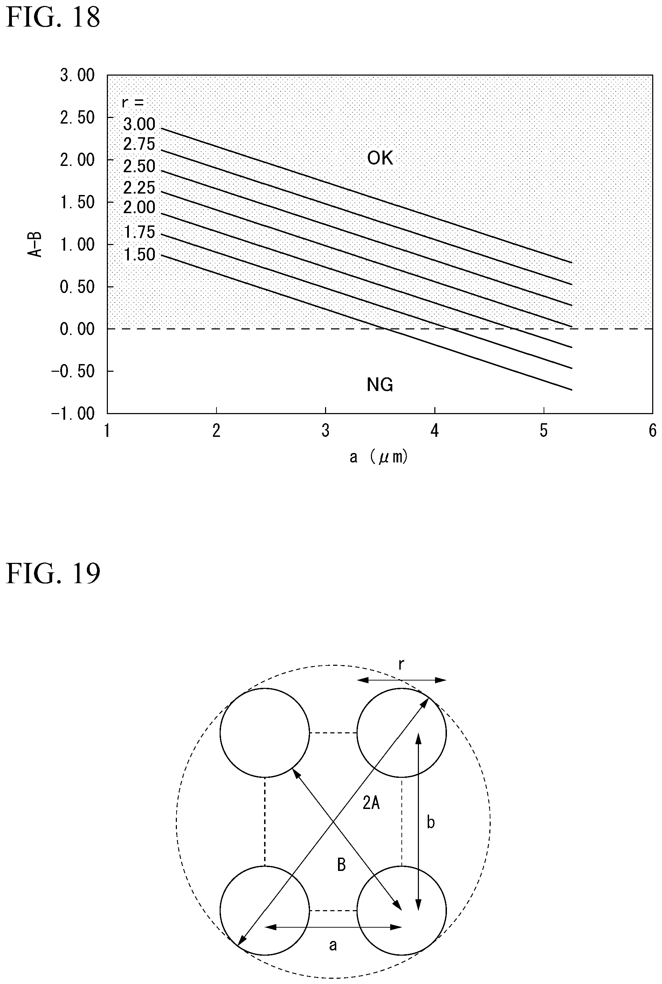

FIG. 18 shows the A-B value when the diameter r of the through-hole 8 is changed to 2.25, 2.50, 2.75, and 3.00 $\mu$m, and the separation distance ($\mu$m) between the centers of the through-holes 8a is changed, that is, the width ($\mu$m) at which the minimum circumscribed circle overlaps the through-hole 8 on the upper side after movement to the center of the lower left through-hole 8.

In FIG. 18, a negative A-B value indicates that the minimum circumscribed circle does not overlap the through-hole 8 on the upper side after movement to the center of the lower left through-hole 8, and is shown as "NG" in the drawing. In FIG. 18, a positive A-B value indicates that the minimum circumscribed circle overlaps the through-hole 8 on the upper side after movement to the center of the lower left through-hole 8, and is shown as "OK" in the drawing. As shown in FIG. 18, for example, when the diameter r of the through-hole 8 ($\mu$m) is 2.25, cases in which the separation distance a ($\mu$m)=3.00 $\mu$m or more and less than 5.32 $\mu$m are examples of the present invention, and cases in which the separation distance a ($\mu$m)=5.32 or more are comparative examples in which requirements of the present invention are not satisfied.

[Experiment 3]

As shown in FIG. 19, conditions for realizing examples were determined when four identical circular through-holes 8 were arranged at the vertex positions of a rectangle. a ($\mu$m) is the separation distance between the centers of the through-holes 8 in the left to right direction in the drawing, b ($\mu$m) is the separation distance between the centers of the through-holes 8 in the up and down direction, r ($\mu$m) is the diameter of the through-hole 8, A ($\mu$m) is the radius of the minimum circumscribed circle that comes into contact with all the through-holes 8, and B ($\mu$m) is the distance from the center of the lower right through-hole 8 to the upper left through-hole 8. The distance B ($\mu$m) is the minimum contact radius of a circle with the center of the minimum circumscribed circle that is moved to the center of the through-hole 8 on the lower right and with the circumference at which the minimum circumscribed circle after the movement is in contact with the through-hole 8 on the upper left.

The radius A ($\mu$m) of the minimum circumscribed circle is calculated using the separation distance a ($\mu$m) in the left to right direction, the separation distance b ($\mu$m) in the up and down direction, and the diameter r ($\mu$m) of the through-hole 8 as follows.

[Math. 6]

$$2A = \sqrt{(a^2 + b^2)} + r$$

The distance B ($\mu$m) from the center of the lower right through-hole 8 to the upper left through-hole 8 is represented by the following formula.

[Math. 7]

$$B = \sqrt{(a^2 + b^2)} - \frac{r}{2}$$

If A>B, since conditions for the present invention are satisfied, the following formula is determined. With the arrangement of FIG. 19, if this formula is satisfied, the effects of the present invention are obtained.

[Math. 8]

$$r > \frac{\sqrt{(a^2 + b^2)}}{2} \quad \Longrightarrow \quad b < \sqrt{4r^2 - a^2}$$

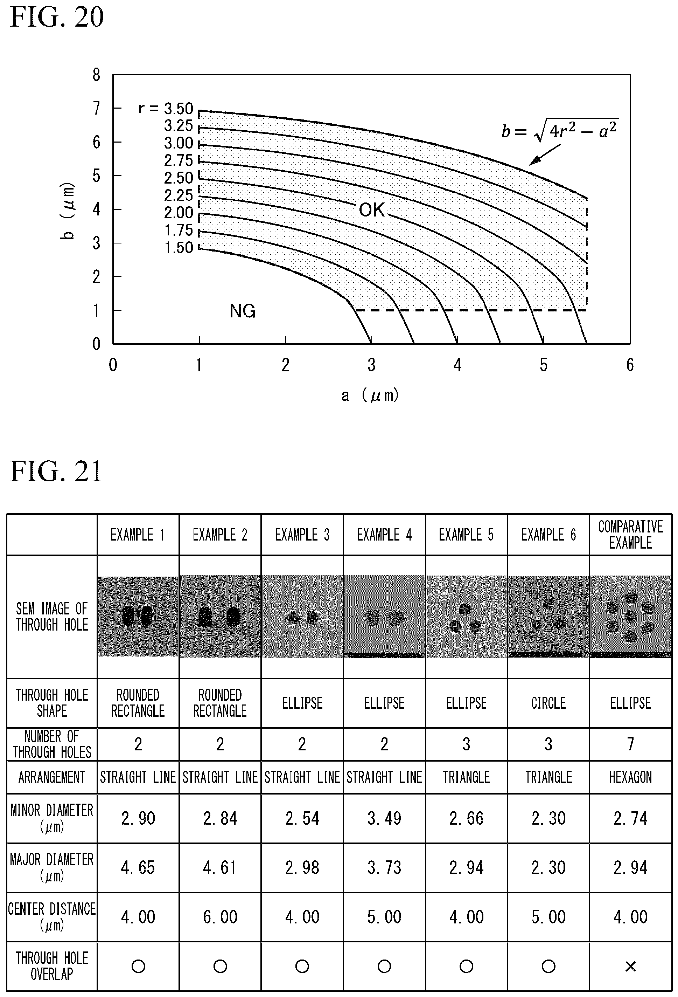

FIG. 20 shows an "OK" range in which conditions for realizing examples of the present invention are satisfied when the separation distance a ($\mu$m) between the centers of the through-holes 8 in the left to right direction, and the separation distance b ($\mu$m) between the centers of the through-holes 8 in the up and down direction are changed for each of cases in which the diameter r of the through-hole 8 ($\mu$m) is set to 1.50, 1.75, 2.00, 2.25, 2.50, 2.75, 3.00, 3.25, and 3.50 $\mu$m. As shown in FIG. 20, it can be understood that, even when the through-holes 8 are arranged at the vertex positions of a rectangle, conditions for realizing examples of the present invention can be easily determined. In the arrangement of FIG. 19, examples of cases in which conditions for examples are satisfied (examples) and cases in which conditions are not satisfied (comparative examples) are shown below.

[Example in which conditions are satisfied]

The separation distance a between the centers of the through-holes 8 in the left to right direction: 4.35 $\mu$m, the separation distance b between the centers of the through-holes 8 in the up and down direction: 4.35 $\mu$m, the diameter r of the through-hole 8: 3.35 $\mu$m, the diameter of the minimum circumscribed circle that comes in contact with all the through-holes 8 (2A): 9.50 $\mu$m

[Example in which conditions are not satisfied]

The separation distance a between the centers of the through-holes 8 in the left to right direction: 4.42 $\mu$m, the

separation distance b between the centers of the through-holes 8 in the up and down direction: 4.54 $\mu$m, the diameter r of the through-hole 8: 3.16 $\mu$m, the diameter of the minimum circumscribed circle that comes in contact with all the through-holes 8 (2A): 9.50 $\mu$m

In addition, when the four rectangular through-holes 8 are arranged with their vertical and horizontal sides aligned on the same line as shown in FIG. 19, specific examples of cases in which conditions for examples are satisfied (examples) and cases in which conditions are not satisfied (comparative examples) are shown below.

[Example in which conditions are satisfied]

The length of the horizontal side of the rectangular through-hole 8: 2.50 $\mu$m, the length of the vertical side of the through-hole 8: 3.25 $\mu$m, the separation distance of the through-hole 8 in the left to right direction: 1.00 $\mu$m, the separation distance of the through-hole 8 in the up and down direction: 1.00 $\mu$m, the diameter of the minimum circumscribed circle that comes in contact with all the through-holes 8: 9.50 $\mu$m

[Example in which conditions are not satisfied]

The length of the horizontal side of the rectangular through-hole 8: 2.00 $\mu$m, the length of the vertical side of the through-hole 8: 2.51 $\mu$m, the separation distance of the through-hole 8 in the left to right direction: 1.00 $\mu$m, the separation distance of the through-hole 8 in the up and down direction: 3.05 $\mu$m, the diameter of the minimum circumscribed circle that comes in contact with all the through-holes 8: 9.50 $\mu$m

[Experiment 4]

A well array filter 1 in which various numbers of through-holes 8 with various shapes and sizes were formed in the well bottom portion 4 was actually prepared, and set in the particle alignment device 40, and a single cell rate after a sample liquid in which cells C were dispersed was passed, and the liquid permeability of ethanol were examined.

Well array filters of Examples 1 to 6 and comparative examples were actually produced by the method described in FIG. 13 as the method of producing a well array filter according to one embodiment. The opening of the well 3 had a regular hexagonal shape, and the opening diameter between two parallel sides was 50 $\mu$m. The partition wall resist 6A was formed from a negative photoresist having a relatively small multifunctional epoxy content, and the bottom portion resist 4A was formed from a negative photoresist having a relatively large multifunctional epoxy content.

FIG. 21 shows scanning electron microscope images (SEM images) of the through-holes 8 in the well array filters produced in Examples 1 to 6 and the comparative example, the shape of each through-hole 8 in a plan view, the number of through-holes 8 in each well 3, the arrangement condition of the through-hole 8, the minor axis ($\mu$m) and the major axis ($\mu$m) of the through-hole 8, the distance ($\mu$m) between the centers of the through-holes 8, and a determination of whether all the through-holes 8 and the minimum circumscribed circle after the movement always overlapped (through-hole overlapping) if the minimum circumscribed circle was moved to the center of any of the through-holes 8. Regarding through-hole overlapping, $\circ$ indicates overlapping, and $\times$ indicates no overlapping.

As shown in FIG. 21, in all of Examples 1 to 6, if the minimum circumscribed circle was moved to the center of any of the through-holes 8, the minimum circumscribed circle after the movement always overlapped all the through-holes 8. On the other hand, in an example in which the through-holes 8 were arranged a the vertex positions of a regular hexagon, when the minimum circumscribed circle was moved to the center of the through-hole 8 on the outer side, the minimum circumscribed circle did not overlap the through-hole 8 on the opposite side.

Next, the well array filters of Examples 1 to 6 and the comparative example were attached to the particle alignment device 40 as a well formation region: 17 mm×17 mm), ethyl alcohol was passed therethrough, and a phosphate buffer (PBS) was then passed three times. Next, 1 ml of a phosphate buffer in which predetermined cells were dispersed in a predetermined number of seeded cells was put into the second chamber 48, and passed through the well array filter. As the cells, peripheral blood mononuclear cells (PBMCs) were used. The number of wells in the well array filter was 90,000, and about 15 to 90% of the number of cells were put into each well.

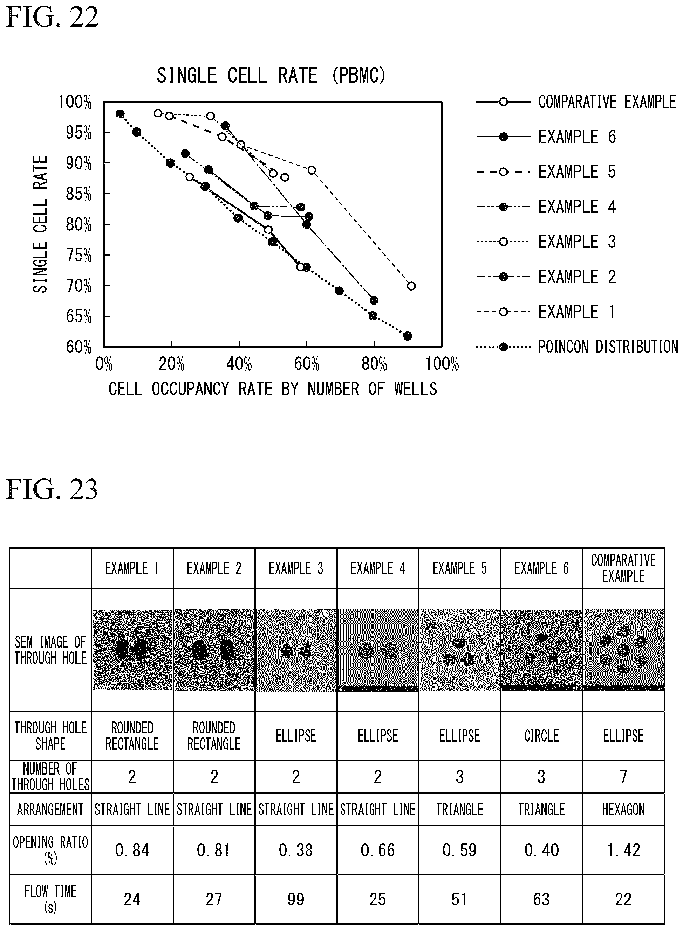

Next, the cells in the wells 3 were stained with an aqueous DAPI solution, the cells in the wells 3 were observed through the bottom plate portion 42 under an inverted fluorescence microscope, and within the field of view of the microscope, among all of the wells, the number of wells A in which one or more cells C were captured and the number of wells B in which only one cell C was captured were measured, and B/A was defined as a single cell rate. The cell occupancy rate (%) relative to the number of wells is a ratio of the number of seeded cells to a total number of wells in the well array filter (the number of seeded cells/the total number of wells). The results are shown in FIG. 22.

In FIG. 22, the numerical values in the "Poisson distribution" indicate the cell occupancy rate (%) relative to the number of wells statistically calculated from the ratio between the number of seeded cells and the number of wells, and the single cell rate (%).

As shown in FIG. 22, in the well array filters of Examples 1 to 6, a single cell rate (%) higher than the single cell rate (%) in the Poisson distribution determined by statistical probability calculation was obtained. This indicates that, after the first cell was captured in the well 3, it became difficult for the second cell to enter the well 3, and the cells flowed into other adjacent wells 3, and indicates that the shape of the well 3 and the settings of the through-hole 8 were appropriate. In Examples 1, 3, and 5 in which the distance between the centers of the through-holes 8 was 4.00 μm, the single cell rate was high. The single cell rate (%) in the comparative example was only about the same as in the case of the Poisson distribution, proving the effects of the present invention.

[Experiment 5]

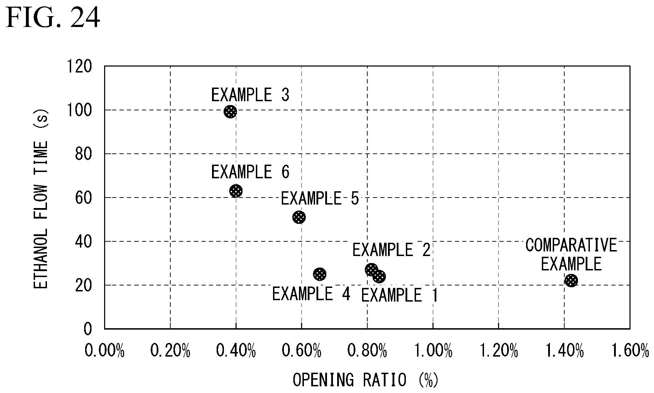

Using the well array filters of Examples 1 to 6 and the comparative example, the time for which ethyl alcohol passed was measured. Each well array filter was attached to the particle alignment device 40, and the well formation region of the well array filter was a 17 mm×17 mm square. 0.5 ml of ethyl alcohol was injected into the second chamber 48, and the time (seconds) required for the entire amount of ethyl alcohol to pass through the well array filter and flow into the flow path 56 was measured. In addition, the ratio of a total opening area of the through-holes 8 relative to the well formation region, that is, the opening ratio (%), was also determined. These results are shown in FIG. 23 and FIG. 24.

In all of Examples 1 to 6, the ethyl alcohol passage time was 99 seconds or shorter, which was within the practical acceptable range. In the comparative example, the opening ratio (%) was high because there were seven through-holes 8, but the liquid passage times in Examples 1, 2, and 4 were comparable to that of the comparative example. Practically, it can be understood that the opening ratio of the through-hole 8 is desirably 0.5% or more because it is easier to use if the ethyl alcohol passage time is 60 seconds or shorter. When the well 3 had a hexagonal shape, the opening diameter between two parallel sides was 10 μm, the thickness of the well partition wall 6 was 2 μm, and circular through-holes with a diameter of 2 μm were provided, thee opening ratio of the through-hole 8 was 3%. Therefore, it can be understood that the opening ratio was desirably in a range of 0.5 to 3%. Based on the results shown in FIGS. 21 to 24, in Examples 1, 2, and 5, both a high single cell rate and liquid permeability were achieved, and Examples 1 and 5 were particularly good.

INDUSTRIAL APPLICABILITY

According to the present invention, since the flow rate of the entire well array filter can be secured while restricting particles to be captured such as cells or beads from passing through through-holed, and the plurality of through-holes formed in each well bottom portion satisfy the positional relationship, when one particle is captured in the well, there is a high probability of the particle at least partially overlapping all of the plurality of through-holes. Therefore, after the first particle is captured, the flow rate of a dispersion medium that passes through all the through-holes in the well is appropriately reduced, the second and subsequent particles are restricted from entering the well according to the flow, and there is a high probability of cells and the like being guided to and captured in an adjacent empty well. Therefore, there is a high probability of particles being captured one by one in many wells, the single cell rate is high, the liquid permeability is favorable, and isolation of particles in each well is stably performed. Therefore, the present invention is industrially applicable.

REFERENCE SIGNS LIST

1 Well array filter
2 Filter body
3 Well
4 Well bottom portion
4A Bottom portion resist
6 Well partition wall
6A Partition wall resist
8 Through-hole
10 Substrate
12 Sacrificial film
14 Gap
40 Particle alignment device
41 Device body
42 Bottom plate portion
44 Wall portion

46 First chamber
48 Second chamber
50 Third chamber
52 Opening
54 Opening
56 Flow path
A Opening diameter
B Partition wall thickness
C Cell
P Pipette
W Separation width
D Diameter
G1 Minimum circumscribed circle
G2 Minimum circumscribed circle after movement
J Overlapping portion

## Claims

1. A well array filter comprising a flat filter body,

   wherein the filter body has a plurality of wells that are formed to open in a well formation region on an upper surface of the filter body, and well partition walls separating the wells adjacent to each other,
   each of the wells has a well bottom portion formed at the lower end of the well, and two or more through-holes are formed in the well bottom portion to reach a lower surface of the filter body,
   the through-hole has an upper-surface opening having a minimum width of 1 $\mu$m or more and 3.5 $\mu$m or less,
   the ratio of a total opening area of the through-holes to the area of the well formation region is 0.5% or more and 3.5% or less, and
   assuming a minimum circumscribed circle that surrounds all of the plurality of through-holes formed in each well bottom portion, when the center of the minimum circumscribed circle is moved to a center of each of the plurality of through-holes, all the minimum circumscribed circles after movement satisfy a positional relationship that the minimum circumscribed circle after movement at least partially overlaps all of the plurality of through-holes.

2. The well array filter according to claim 1, wherein the through holes formed in each of the well bottom portions are formed such that:

   two or more through-holes are formed on an imaginary line segment; or
   a total of three or more through-holes are formed at vertex positions of an imaginary triangle, or on the sides or inside of the imaginary triangle; or
   a total of four or more through-holes are formed at vertex positions of an imaginary quadrangle, or on the sides or inside of the imaginary quadrangle.

3. The well array filter according to claim 1 or 2,
   wherein the opening shape of the through-hole is a circle, an ellipse, or a shape in which a plurality of circles or ellipses are connected.

4. The well array filter according to claim 1 or 2,
   wherein the opening shape of the through-hole is a polygonal shape or a polygonal shape with rounded corners.

5. The well array filter according to claim 1 or 2,
   wherein a polymer that restricts cell adhesion is applied to at least the inner surface of the well.

6. A particle alignment device, comprising the well array filter according to claim 1 or 2, and a device body that supports the well array filter and has a sample flow path from the side of the upper-surface opening of the well of the well array filter toward the lower surface of the well bottom portion through the through-hole.

7. A particle capture method, comprising:

a step of passing a liquid containing an organic solvent that does not dissolve or alter the well array filter and the device body through the sample flow path in the particle alignment device according to claim 6;

a step of passing an aqueous solution through the sample flow path and replacing the liquid containing the organic solvent with the aqueous solution; and

a step of passing an aqueous solution containing particles to be captured in the well through the sample flow path and capturing the particle in the well.

## FIG. 1

## FIG. 2

FIG. 3

FIG. 4

FIG. 5

FIG. 6

（a）

（b）

（c）

FIG. 7

FIG. 8

(a)

(b)

(c)

FIG. 9

(a)

(b)

(c)

FIG. 10

FIG. 11

FIG. 12

FIG. 13

( a )
12
10

⇓

( b )
4A
12
10

⇓

( c )
8  14  8  14  8  4
12
10

⇓

( d )
4  8  14  8  14  8  6A
4
12
10

⇓

( e )
6  6  6  6
3  3  3
8  4  8  4  8  4
12
10

⇓

( f )
6  6  6  6
3  3  3
1
8  4  8  4  8  4
2

FIG. 14

FIG. 15

FIG. 16

FIG. 17

FIG. 18

FIG. 19

## FIG. 20

$$b = \sqrt{4r^2 - a^2}$$

## FIG. 21

| | EXAMPLE 1 | EXAMPLE 2 | EXAMPLE 3 | EXAMPLE 4 | EXAMPLE 5 | EXAMPLE 6 | COMPARATIVE EXAMPLE |
|---|---|---|---|---|---|---|---|
| SEM IMAGE OF THROUGH HOLE | | | | | | | |
| THROUGH HOLE SHAPE | ROUNDED RECTANGLE | ROUNDED RECTANGLE | ELLIPSE | ELLIPSE | ELLIPSE | CIRCLE | ELLIPSE |
| NUMBER OF THROUGH HOLES | 2 | 2 | 2 | 2 | 3 | 3 | 7 |
| ARRANGEMENT | STRAIGHT LINE | STRAIGHT LINE | STRAIGHT LINE | STRAIGHT LINE | TRIANGLE | TRIANGLE | HEXAGON |
| MINOR DIAMETER ($\mu$m) | 2.90 | 2.84 | 2.54 | 3.49 | 2.66 | 2.30 | 2.74 |
| MAJOR DIAMETER ($\mu$m) | 4.65 | 4.61 | 2.98 | 3.73 | 2.94 | 2.30 | 2.94 |
| CENTER DISTANCE ($\mu$m) | 4.00 | 6.00 | 4.00 | 5.00 | 4.00 | 5.00 | 4.00 |
| THROUGH HOLE OVERLAP | ○ | ○ | ○ | ○ | ○ | ○ | × |

## FIG. 22

### SINGLE CELL RATE (PBMC)

Legend:
- COMPARATIVE EXAMPLE
- EXAMPLE 6
- EXAMPLE 5
- EXAMPLE 4
- EXAMPLE 3
- EXAMPLE 2
- EXAMPLE 1
- POINCON DISTRIBUTION

X-axis: CELL OCCUPANCY RATE BY NUMBER OF WELLS
Y-axis: SINGLE CELL RATE

## FIG. 23

| | EXAMPLE 1 | EXAMPLE 2 | EXAMPLE 3 | EXAMPLE 4 | EXAMPLE 5 | EXAMPLE 6 | COMPARATIVE EXAMPLE |
|---|---|---|---|---|---|---|---|
| SEM IMAGE OF THROUGH HOLE | | | | | | | |
| THROUGH HOLE SHAPE | ROUNDED RECTANGLE | ROUNDED RECTANGLE | ELLIPSE | ELLIPSE | ELLIPSE | CIRCLE | ELLIPSE |
| NUMBER OF THROUGH HOLES | 2 | 2 | 2 | 2 | 3 | 3 | 7 |
| ARRANGEMENT | STRAIGHT LINE | STRAIGHT LINE | STRAIGHT LINE | STRAIGHT LINE | TRIANGLE | TRIANGLE | HEXAGON |
| OPENING RATIO (%) | 0.84 | 0.81 | 0.38 | 0.66 | 0.59 | 0.40 | 1.42 |
| FLOW TIME (s) | 24 | 27 | 99 | 25 | 51 | 63 | 22 |

FIG. 24

# INTERNATIONAL SEARCH REPORT

International application No.

**PCT/JP2023/037068**

| A. | CLASSIFICATION OF SUBJECT MATTER |
|---|---|

***B01D 29/01***(2006.01)i; ***C12N 1/00***(2006.01)i; ***C12M 1/28***(2006.01)i
FI:   B01D29/04 510Z; B01D29/04 510A; C12M1/28; C12N1/00 Z

According to International Patent Classification (IPC) or to both national classification and IPC

| B. | FIELDS SEARCHED |
|---|---|

Minimum documentation searched (classification system followed by classification symbols)

B01D24/00-35/05;35/10-37/04; C12N1/00-7/08; C12M1/00-3/10; G01N35/00-37/00; G01N1/00-1/44

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2021/241283 A1 (TOKYO OHKA KOGYO COMPANY, LIMITED) 02 December 2021 (2021-12-02)<br>     paragraphs [0040]-[0043], fig. 8 | 1-7 |
| A | WO 2021/065765 A1 (TOKYO OHKA KOGYO COMPANY, LIMITED) 08 April 2021 (2021-04-08)<br>     paragraphs [0020], [0036]-[0040], fig. 8 | 1-7 |
| A | JP 2022-536796 A (GEORGIA TECH. RESEARCH CORPORATION) 18 August 2022 (2022-08-18)<br>     paragraph [0120] | 5 |
| A | JP 2015-203582 A (NAGOYA UNIVERSITY) 16 November 2015 (2015-11-16)<br>     entire text, all drawings | 1-2 |
| A | JP 2018-061447 A (HITACHI CHEMICAL COMPANY, LIMITED) 19 April 2018 (2018-04-19)<br>     paragraph [0036] | 3-4 |

✓ Further documents are listed in the continuation of Box C.          ✓ See patent family annex.

| | |
|---|---|
| *     Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **20 December 2023** | **09 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2023/037068** |

## C. DOCUMENTS CONSIDERED TO BE RELEVANT

| Category* | Citation of document, with indication, where appropriate, of the relevant passages | Relevant to claim No. |
| --- | --- | --- |
| A | WO 2021/262572 A1 (CORNING INCORPORATED) 30 December 2021 (2021-12-30) paragraph [0025] | 7 |

Form PCT/ISA/210 (second sheet) (January 2015)

**INTERNATIONAL SEARCH REPORT**
Information on patent family members

International application No.

**PCT/JP2023/037068**

| Patent document cited in search report | | | Publication date (day/month/year) | Patent family member(s) | | | Publication date (day/month/year) |
|---|---|---|---|---|---|---|---|
| WO | 2021/241283 | A1 | 02 December 2021 | US 2023/0173487 A1 paragraphs [0058]-[0061], fig. 8 | | | |
| | | | | EP 4159837 | A1 | | |
| | | | | CN 115698254 | A | | |
| WO | 2021/065765 | A1 | 08 April 2021 | US 2022/0340859 A1 paragraphs [0030], [0046]-[0054], fig. 8 | | | |
| | | | | EP 4040164 | A1 | | |
| | | | | CN 114466919 | A | | |
| | | | | KR 10-2022-0070217 | A | | |
| JP | 2022-536796 | A | 18 August 2022 | US 2022/0298489 A1 paragraph [0140] | | | |
| | | | | WO 2020/257247 | A1 | | |
| | | | | CN 114599781 | A | | |
| | | | | CA 3143985 | A1 | | |
| JP | 2015-203582 | A | 16 November 2015 | US 2017/0122937 A1 entire text, all drawings | | | |
| | | | | WO 2015/156343 | A1 | | |
| | | | | EP 3130905 | A1 | | |
| JP | 2018-061447 | A | 19 April 2018 | (Family: none) | | | |
| WO | 2021/262572 | A1 | 30 December 2021 | JP 2023-532649 A paragraph [0020] | | | |
| | | | | US 2023/0235264 | A1 | | |
| | | | | CN 115956116 | A | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022181802 A
- US 10370630 B
- US 9638636 B
- JP 2019213566 A